# EUROPEAN PATENT SPECIFICATION

(11) **EP 0 502 119 B1**
(45) Date of publication and mention of the grant of the patent: **31.01.1996**
(21) Application number: 91901106.4
(22) Date of filing: 08.11.1990
(51) Int. Cl.: A61K 9/16, A61K 9/50, A01N 25/26

(54) **LIPOSPHERES FOR CONTROLLED DELIVERY OF SUBSTANCES**
LIPOSPHÄREN ZUR KONTROLLIERTEN SUBSTANZABGABE
LIPOSPHERES D'ADMINISTRATION REGULEE DE SUBSTANCES

(30) Priority: 13.11.1989 US 435546
(43) Date of publication of application: 09.09.1992
(73) Proprietor: SCIOS NOVA, INC., Baltimore, MD 21224-6522 (US)
(72) Inventor: DOMB, Abraham, J., Baltimore, MD 21209 (US); MANIAR, Manoj, Baltimore, MD 21237 (US)
(74) Representative: Bassett, Richard Simon
(86) International application number: US9006519
(87) International publication number: WO9107171

(56) References cited:
- EP-A- 0 042 249
- EP-A- 0 167 825
- EP-A- 0 177 368
- EP-A- 0 209 870
- EP-A- 0 270 460
- EP-A- 0 274 431
- WO-A-85/00011
- US-A- 3 804 776
- US-A- 4 332 796

## Description

### Background of the Invention

This invention is in the area of controlled delivery systems for substances, including pharmaceuticals, vaccines, and insect control agents.

Many dispersion systems are currently in use as, or being explored for use as, carriers of substances, particularly biologically active compounds. These systems are designed to protect the substance from the environment during delivery and to provide a controlled release of the substance to a targeted area. In some cases, the goal is to target specific sites in the body using the dispersion. In other cases, the goal is to prepare a drug carrier system that acts as a reservoir at the site of injection. Dispersed systems for delivery are also important in the non-pharmaceutical area, for example, for use in controlled release of substances for insect control and agricultural applications.

Dispersion systems used for pharmaceutical and cosmetic formulations can be categorized as either suspensions or emulsions. Suspensions are defined as solid particles ranging in size from a few nanometers up to hundreds of microns, dispersed in a liquid medium using suspending agents. Solid particles include microspheres, microcapsules, and nanospheres.

Emulsions can be defined as dispersions of one liquid in another, stabilized by an interfacial film of emulsifiers such as surfactants and lipids. Despite their long history, emulsions are used less often today than many other dosage forms due to the inherent instability. Emulsion formulations include water in oil and oil in water emulsions, multiple water/oil/water emulsions, microemulsions, microdroplets, and liposomes.

Microdroplets are unilamellar phospholipid vesicles that consist of a spherical lipid layer with an oil phase inside, as defined in U.S. Patent Nos. 4,622,219 and 4,725,442 issued to Haynes. Liposomes are phospholipid vesicles prepared by mixing water-insoluble polar lipids with an aqueous solution. The unfavorable entropy caused by mixing the insoluble lipid in the water produces a highly ordered assembly of concentric closed membranes of phospholipid with entrapped aqueous solution. The membrane consists of a bimolecular sheet of lipid molecules that have their hydrophobic centers aligned in the middle of the membrane, and hydrophilic ends on the outside of the membrane, interfacing with the aqueous solution, and on the inside, interfacing with entrapped aqueous solution. A unilamellar liposome has a single membrane encapsulating a solution. A multilamellar liposome has a series of membranes, each of which is separated from the neighboring membrane by water. Unilamellar liposomes have a minimum size of about 25 nanometers in diameter, while multilamellar liposomes can range up to several micrometers. The rigidity and permeability of phospholipid bilayers can be adjusted by including other water-insoluble components such as sterols and amphiphiles with the phospholipid. Emulsion based delivery systems are subject to physical rupture because of the delicate nature of the liquid/membrane/liquid structure. Emulsion based delivery systems also have relatively short release times. Further, it is difficult to isolate emulsion based vesicles from the aqueous media used for storage for subsequent reconstitution.

The solid phase vesicles in suspension based delivery systems are more stable than the liquid vesicles in emulsion based delivery systems. However, in suspension based systems the concentration of the suspension and the interaction between the vesicles and the liquid carrier is limited because of the repulsive interaction between the solid and the carrier.

Few dispersion systems can be used for delivery and release of a variety of compounds, both in vivo and as applied to plants and the environment. Areas of particular interest include delivery of drugs, such as anesthetics, immunization, particularly using antigens that normally do not elicit a protective immunological response, and pesticide control, especially insect repellents.

Products for the moderation of pain, referred to as analgesics, represent one of the largest markets targeted by pharmaceutical companies. Pharmaceutical analgesics include a variety of classes of drugs, such as general anesthetics, non-steroidal anti-inflammatories, and local anesthetics. General anesthetics reduce pain by producing a loss of consciousness. Local anesthetics cause a loss of sensation in a localized area of the body without a loss of consciousness. Non-steroidal anti-inflammatories may ameliorate the pain but do not cause a loss of sensation or consciousness. Among the general anesthetics are centrally acting narcotics, including morphine, demerol, fentanyl and codeine. These drugs act through opiate receptors in the central nervous system. Non-steroidal anti-inflammatory drugs (NSAIDs) include ibuprofen, indomethacin, acetaminophen, piroxicam, naproxen, flufenamic acid and mefenamic acid. Local anesthetics block the generation and conduction of nerve impulses by increasing the threshold for electrical excitation in the appropriate nerve, by slowing the propagation of the nerve impulse, and by reducing the rate of rise of the action potential. Local anesthetics are extremely potent and result in a virtually complete loss of sensation in the treated area of the body.

Local anesthetics are preferred over general anesthetics because of the serious complications that can occur during general anesthesia. However, even local anesthetics, which are usually injected as an aqueous solution, are eventually absorbed from the site of application into the circulation system, and their therapeutic indices, dosages, and frequency of dosage, must therefore be strongly considered before administration to a patient. The more frequently tee local anesthetic must be administered, the more likely it is that systemic toxicity will develop.

Although a number of delivery systems have been utilized for administration of anesthetics, all have limitations with respect to cost and ease of manufacture, stability, length of effectiveness, and utility with a variety of anesthetics.

An immune response can be induced against an almost limitless variety of substances. There are two principal types of immune responses: cell mediated responses and humoral responses. In both situations, an antigen, or foreign substance, is recognized by specific receptors on the surfaces of lymphocytes. Humoral responses are characterized by the production of antibodies specifically directed against regions of the antigen known as epitopes. Under normal circumstances, antigen recognition results in the destruction and/or removal of the antigen from the animal.

Induction of antibody (Ab) formation and of specific cellular responses is referred to as immunization even when infectious agents are not involved. Vaccination usually refers to immunization in which a suspension of infectious agents (or parts thereof) is administered to an animal to induce resistance to the disease caused by the infectious agents.

The intentional vaccination of individuals to protect against disease began in the eighteenth century with the inoculation of scrapings from persons infected with cowpox to protect against the related disease smallpox. Techniques have improved substantially since that time, leading to a decreased incidence of side effects due to reactions against contaminants in the vaccine and to the elicitation of an immunogenic response against materials not normally eliciting an immune response. These enhanced effects have been achieved by altering the antigen by attaching it to a carrier molecule, by chemical modification, and by administering it in conjunction with a vehicle increasing the immunological response to the antigen (referred to as an adjuvant).

Many infectious agents have proven difficult, if not impossible, to elicit an immunological response to that is effective in preventing subsequent infection by the agent. Other agents, such as toxins and agents used in biological and chemical warfare, are not immunogenic in the form to which a person or other animal would be exposed. While many delivery systems have been used to increase the immunological response to these poor antigens, especially liposomes, they are limited by cost, stability, and limited effectiveness.

Insects such as mosquitos, flies and fleas are a significant factor in the spread of many serious diseases of man and other animals. They are also major pests, annoying humans and animals, leading to lost work and foraging time. Efforts to repel these insect pests, other than with physical means, are generally ineffective for more than a few hours.

Most means for repelling insects consist of applying an organic compound such as N,N-diethyl-m-toluamide (DEET) to the skin in an organic solvent. DEET is the most effective compound known to repel mosquitos, however, even its effectiveness is limited to a few hours. Moreover, the solvent in which it is applied to the skin also facilitates passage of the DEET through the skin, leading to undesirable toxicity, as well as irritation of the skin and eyes. A variety of other compounds have been used to repel or kill other insects, especially flies and fleas. Most flea repellents or insecticides consist of a combination of active ingredients, many of which are not stable over a prolonged period of time. Efforts to extend the period of effectiveness have not met with great success, although some microencapsulated forms are being marketed. These consist either of polymer or protein microcapsules incorporating the active agent to be released. The disadvantages of these reagents are that they are expensive to manufacture, they contain polymer residuals, and they are gritty and uncomfortable when applied to the skin.

It is therefore an object of the present invention to provide a controlled delivery system for substances that is stable for an extended time.

It is another object of the present invention to provide a controlled delivery device that has a relatively long release time.

It is still another object of this invention to provide a suspension delivery vesicle that can be isolated for storage and then resuspended when desired.

It is an object of the present invention to provide a composition and method to locally alleviate pain for an extended period without the need for frequent administration of the drug.

It is a further object of the present invention to provide a composition and method to locally alleviate pain for an extended period without the side effects associated with general anesthetic.

It is another object of the present invention to provide a composition, and method of use thereof, for alleviation of pain, that is easy to prepare and stable for an extended period of time prior to use and in vivo.

It is an object of the present invention to provide a composition and method to administer antigens for immunization of an animal.

It is a further object of the present invention to provide a composition and method to effect or enhance immunization of an animal against an antigen that does not normally produce a good immunological response.

It is another object of the present invention to provide a composition, and method of use thereof, for immunization of an animal against an antigen that is easy to prepare and stable for an extended period of time prior to use and in vivo.

It is an object of the present invention to provide a composition and method to release insect repellents over an extended period of time, especially on the skin of a human or other animal.

It is a further object of the present invention to provide a composition and method to release insect repellents with minimum absorption through the skin or release of irritating chemicals at the application site.

It is another object of the present invention to provide a composition, and method of use thereof, for release of insect repellents, that is easy to prepare and stable for an extended period of time prior to use and in vivo.

### Summary of the Invention

Solid, water-insoluble lipospheres formed of a solid hydrophobic core having a layer of a phospholipid embedded on the surface of the core, containing biologically active agent in the core, in the phospholipid, adhered to the phospholipid, or a combination thereof, are disclosed for use in providing extended release of active agent, including drugs such as vaccines and anesthetics, insect control agents such as insecticides and repellents, and agricultural compounds such as fertilizers, fungicides, pesticides, and herbicides.

Lipospheres can be prepared by a melt technique or a solvent technique, summarized as: (1) forming a liquid solution or suspension of the agent by either melting the agent, or dissolving or dispersing the agent in a liquid vehicle, to form a mixture of liquid agent that solidifies at room temperature or greater; (2) adding phospholipid and an aqueous solution to the liquid agent to form a suspension; (3) mixing the suspension at a temperature above the melting temperature until a homogeneous fine dispersion is obtained; and then (4) rapidly cooling the dispersion to below the melting temperature of the liquid mixture containing the agent. Agent can also be added to the phospholipid or mixed with the resulting lipospheres.

The agent containing lipospheres have several advantages over other delivery systems, including emulsions, vesicles and liposomes, including stability, low cost of reagents, ease of manufacture, high dispersibility in an aqueous medium, a release rate for the entrapped substance that is controlled by the phospholipid coating and the carrier.

Examples demonstrate the effective administration of anesthetics, antigens, and insect repellent (DEET).

### Brief Description of the Drawings

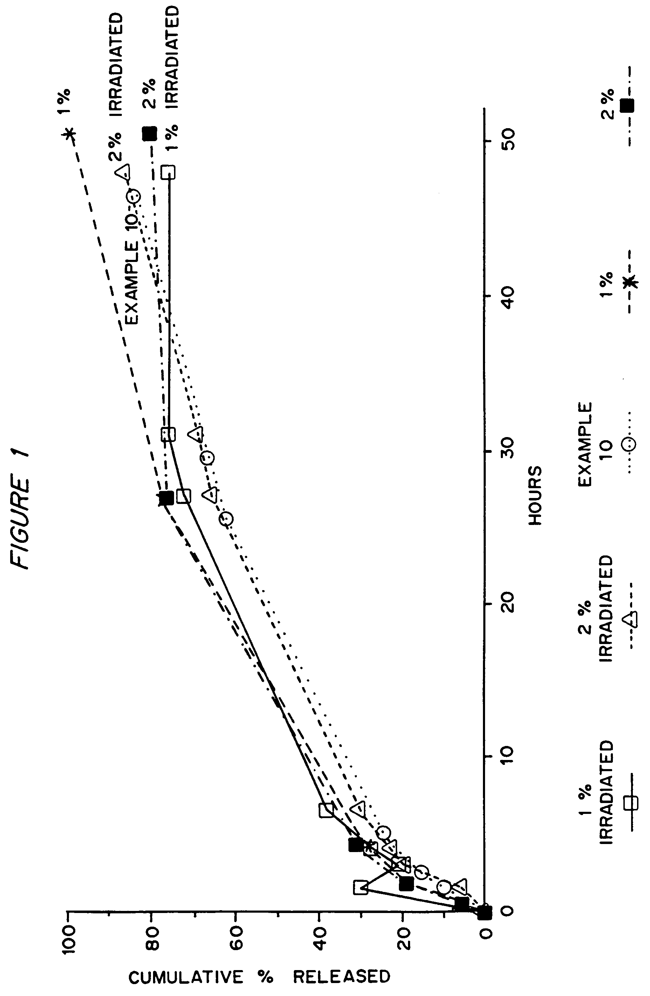

Figure 1 is graph of the release (cumulative % released) over time (hours) of marcaine from lipospheres formed of marcaine:tristearin:phosphatidyl choline, 1:4:2 (-*-), (-square-); and 2:4:2 (-dark square-), (-triangle-), in suspension and after irradiation, respectively. The release of marcaine from lipospheres made by sonication (-O-) is also shown.

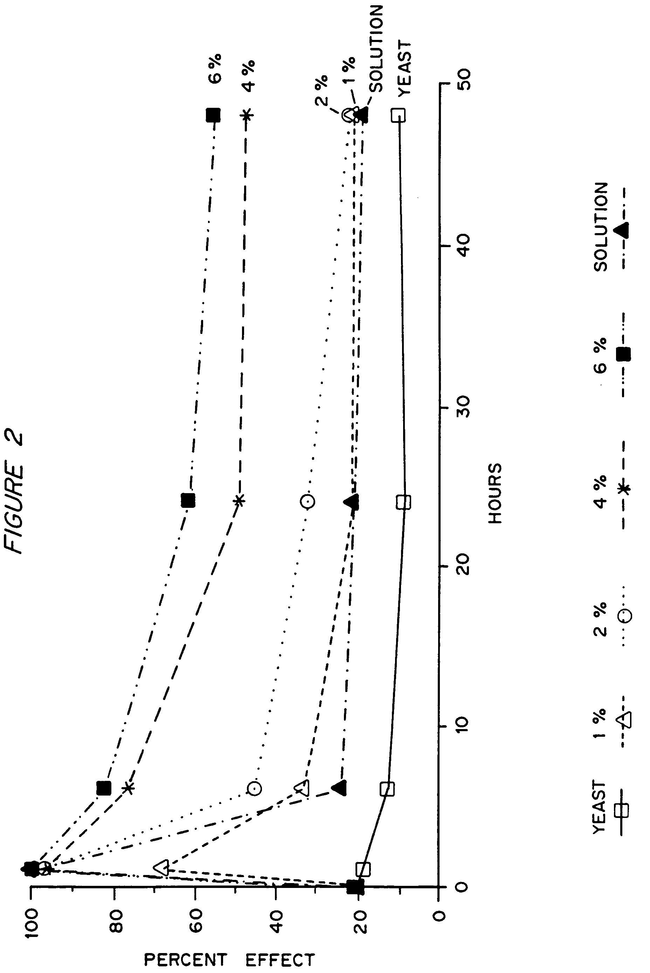

Figure 2 is a graph of the in vivo percent effect over time (hours) of marcaine from lipospheres having loadings of 1% (-triangle-), 2% (-O-), 4% (-*-), and 6% (-dark square-), compared with a yeast control (-square-) and 1% marcaine HCl solution (-dark triangle-).

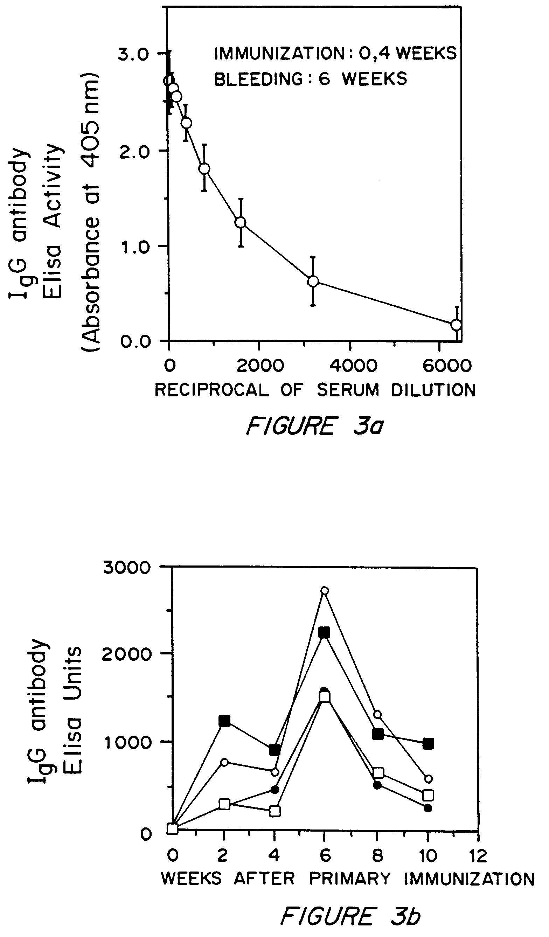

Figure 3A is a graph of the IgG antibody (ELISA units) against [M(R32NS1 + LA) + Alum] over time following immunization (weeks) for four rabbits immunized intramuscularly at 0 and 4 weeks with 0.5 to 1 ml lipospheres containing 100 µg R32NS1 malaria antigen and 40 µg lipid A adsorbed with alum. Figure 3B is a graph of the mean IgG antibody levels (ELISA activity based on absorbance at 405 nm) for the four rabbits six weeks after immunization with lipospheres containing the R32NS1 antigen as function of the reciprocal of serum dilution.

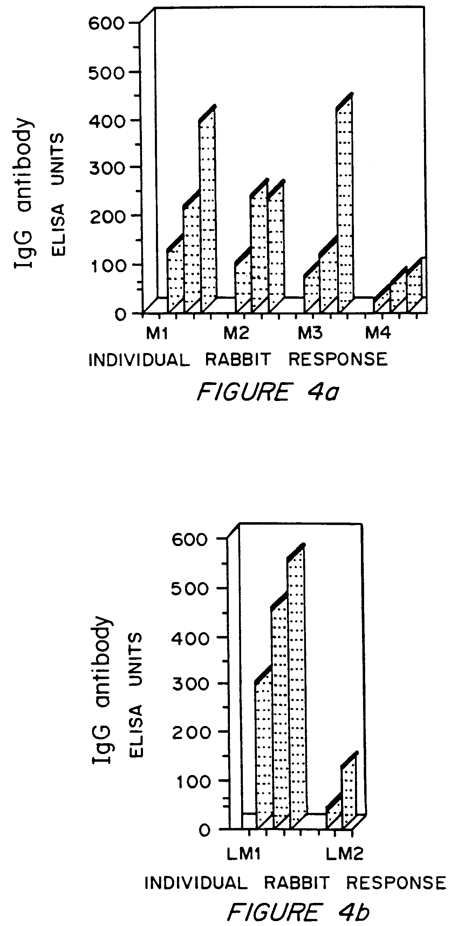

Figure 4A is a graph of the antibody produced (ELISA units) six weeks after immunization (at zero time and again four weeks later) with liposphere formulations (between 0.5 and 1 ml) containing malarial antigens, M1 and M2, containing lipid A, (10 µg) or M3 and M4 lacking lipid A (10 µg), where the antigen was incorporated in the lipospheres from an aqueous buffer solution (M1) or added as a lyophilized powder to the lipid components in the first step of liposphere preparation (M3), at R32NS1 antigen doses of 100 µg adsorbed with alum (1 mg/ml final concentration).

Figure 4B is a graph of the antibody produced (ELISA units) six weeks after immunization (at zero time and again four weeks later) with between 0.5 to 1 ml of lipospheres containing the malaria antigen R32NS1 (100 µg) and lipid A (LM1, 20 µg) or lacking lipid A (LM2).

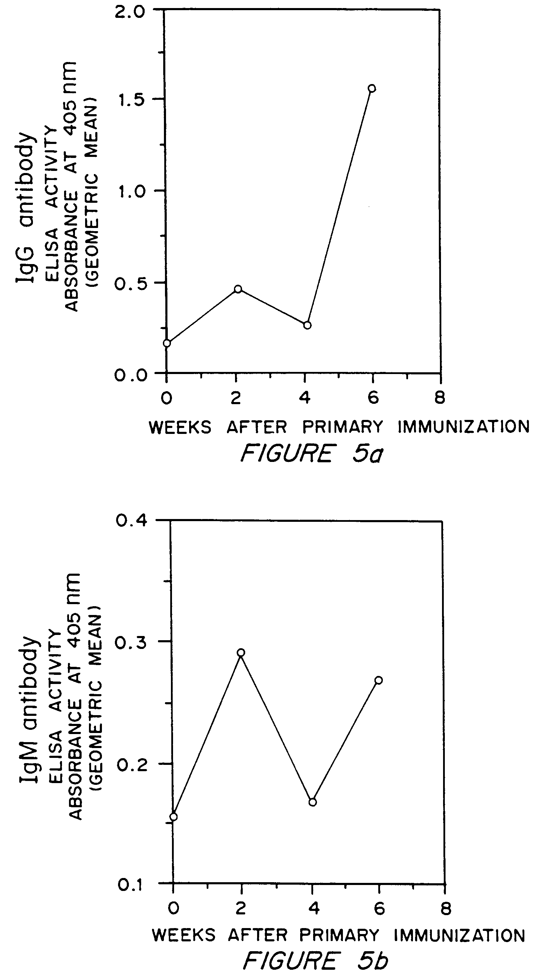

Figure 5 are graphs of the antibody produced (ELISA units) as a function of the time (weeks) after immunization (at zero time and again four weeks later) with liposphere formulations containing lipid A (40 µg) and R32NS1 (100 µg) for IgG antibodies (Figure 5A) and IgM antibodies (Figure 5B) to lipid A.

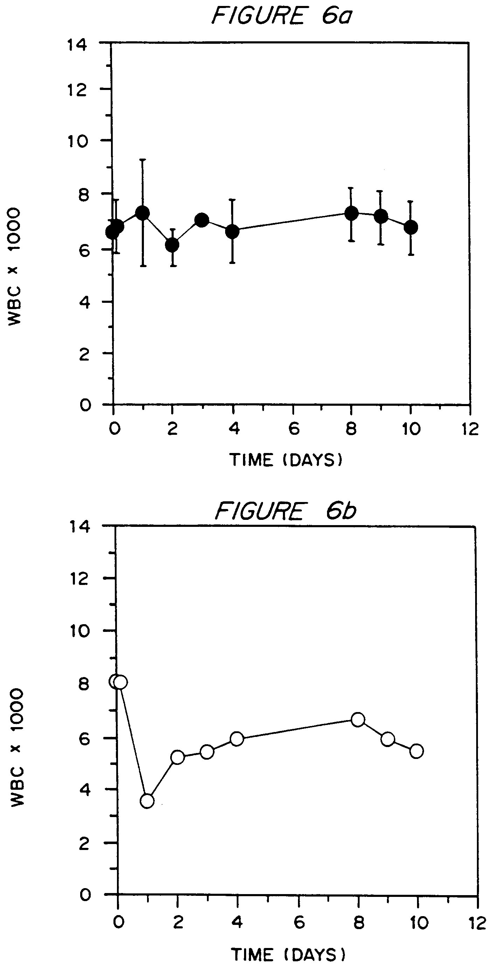

Figure 6 are graphs of the white blood cell (WBC) count (x1000) over time (days) of rabbits injected intramuscularly with lipospheres containing R32NS1 (125 µg/dose) and lipid A (60 µg/dose) (Figure 6A) or free lipid A (60 µg) (Figure 6B) over time (days).

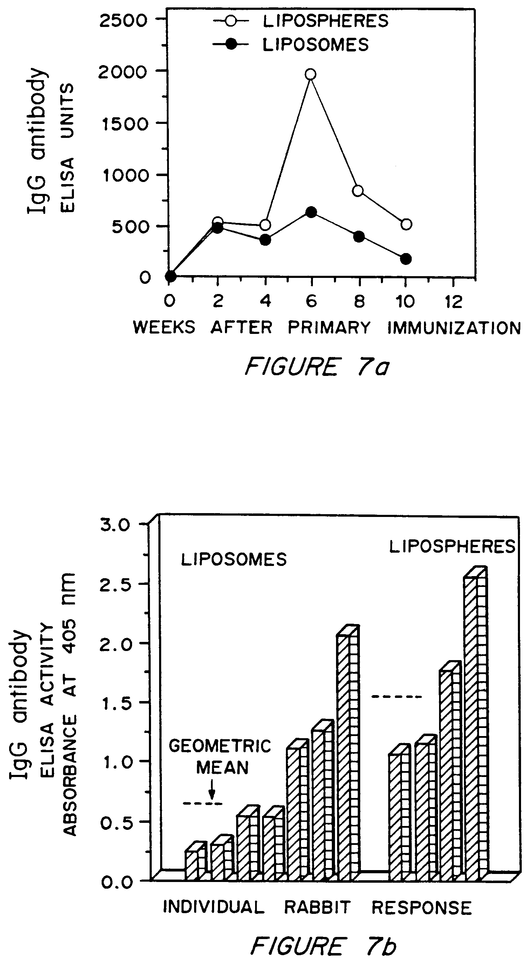

Figure 7 are graphs of the antibody produced (ELISA units) as a function of the time (weeks) after immunization (at zero time and again four weeks later) with liposphere formulations containing lipid A and a malarial antigen (R32tet SKB) in combination with alum (Figure 7A, mean titer for group, --*--) or liposomes containing lipid A and the malarial antigen in combination with alum (Figure 7A, mean titer for group, - - * - -). The individual responses are shown in Figure 7B. The mean antibody titer for each of the rabbits in the two groups is shown in Figure 7A.

### Detailed Description of The Invention

Lipospheres consist of an inner core that is a solid at approximately room temperature or greater surrounded by a phospholipid layer embedded in the solid core. A substance to be delivered (or released) can be incorporated into the core, the phospholipid surface layer, adhered to the outer phospholipid layer, or a combination thereof. Any number of substances can be incorporated, including biologically active agents such as pharmaceuticals for enteral, parenteral or topical administration to humans or other animals, agents for pest control such as insect repellents and insecticides, and agents for agricultural applications such as herbicides, fungicides, and fertilizers.

The lipospheres are distinct from microdroplets, vesicles or liposomes since the lipospheres have solid inner cores at room temperature. The lipospheres are distinct from microspheres of uniformly dispersed material in homogenous polymer since they consist of at least two layers, the inner solid particle and the outer layer of phospholipid.

The combination of solid inner core with phospholipid exterior confers several advantages on the lipospheres as compared with conventional microspheres and microparticles, including high dispersibility in an aqueous medium, and a release rate for the entrapped substance that is controlled by the phospholipid coating and the carrier. There are also many advantages over other dispersion based delivery systems. Lipospheres have increased stability as compared to emulsion based systems, including vesicles and liposomes, and are more effectively dispersed than most suspension based systems. Further, the substance to be delivered does not have to be soluble in the vehicle since it can be dispersed in the solid carrier. Lipospheres also have a lower risk of reaction of substance to be delivered with the vehicle than in emulsion systems because the vehicle is a solid inert material. Moreover, the release rate of the substance from the lipospheres can be manipulated by altering either or both the inner solid vehicle or the outer phospholipid layer. Lipospheres are also easier to prepare than vehicles such as lipospheres, and are inherently more stable. Stability has become the major problem limiting the use of liposomes, both in terms of shelf life and after administration in vivo. Liposomes and vesicles do not remain intact or available in vivo after injection for more than a few hours to a couple of days. Unlike many of the biodegradable polymeric systems, the lipospheres not made with biodegradable polymers are stable in aqueous solutions. As importantly, the cost of the reagents for making the lipospheres (food grade) is significantly less than the cost of reagents for making liposomes, which require very pure lipids. For example, food grade lecithin costs about $2 per pound (from central Soya Co., Ft. Wayne, IN), as compared with liposome grade lecithin which costs about $500 per pound (Avanti Polar Lipids Inc., Pelham, AL).

### Preparation of Lipospheres

The preparation and modification of lipospheres is described first with reference to general descriptions and then with reference to examples of the preparation and application of lipospheres.

### Selection of the Solid Core of the Liposphere.

In the preferred embodiment, the liposphere contains a core that has a melting temperature equal to or greater than room temperature, approximately 25°C. For example, for preparation of lipospheres to be applied to the skin, the core is prepared by choosing an agent to be delivered or released that has a melting temperature of approximately 30°C, or by mixing the agent in a carrier to produce a mixture having a melting point of approximately 30°C. The agent, or agent and carrier, preferably has a melting point of less than 120°C and is stable in the liquid form when mixed with hot aqueous media.

The carrier must be compatible with the agent to be delivered or released. Suitable solid carriers are inert hydrophobic biocompatible materials with a melting range between 30° and 120°C. Examples are natural, regenerated, or synthetic waxes such as beeswax and carnauba wax; cholesterol; fatty acid esters such as ethyl stearate, isopropyl myristate, and isopropyl palmitate; high molecular weight fatty alcohols such as cetostearyl alcohol, cetyl alcohol, stearyl alcohol, and oleyl alcohol; solid hydrogenated castor and vegetable oils; hard and soft paraffins; hard fat such as tristearin; biodegradable polymers such as polycaprolactone, polyamides, polyanhydrides, polycarbonates, polyorthoesters, polylactic acids, and copolymers of lactic acid and glycolic acid; cellulose derivatives and mixtures thereof. These and other suitable materials are known to those skilled in the art and most are commercially available, as demonstrated by the extensive list of suitable carrier materials in Martindale, The Extra Pharmacopoeia, The Pharmaceutical Press, 28th Edition pp 1063-1072 (1982).

The release rate of the agent from the liposphere is dependent in part upon the composition of the core, as well as the outer phospholipid layer, and can be altered by varying the compositions appropriately.

It is sometimes desireable to deliver a water soluble agent to a targeted area, or to control the release of a water soluble substance. Since the inner core of the liposphere is hydrophobic, it is necessary to decrease the water solubility of the agent before liposphere preparation. Methods to decrease water solubility include using a water insoluble salt or base, complex, or insoluble precursor form of the agent; preincorporating the agent into hydrophobic microparticles that can be used as agent particles; or preparing an aqueous medium that the agent is less soluble in, for example, by adjustment of pH or ionic strength, or by adding salts or additives. If the agent is rendered less water soluble by adjustment of pH or ionic strength, the resulting lipospheres can be isolated by filtration or centrifugation and reconstituted with an appropriate buffer solution prior to use.

Active materials can be preincorporated into microparticles of a hydrophobic solid phase, such as tristearin (melting point 65°C to 72°C), that can then be incorporated into lipospheres with a vehicle having a lower melting point, such as ethyl stearate (melting point 35°C), to avoid melting the tristearin particles containing the active material. In this form, the tristearin-active material particles are the hydrophobic "agent" which is dispersed in the ethyl stearate liposphere. The formulations can then be freeze dried with standard techniques and reconstituted prior to use.

### Selection of the Phospholipid Coating.

The solid core of the liposphere is coated with one or more phospholipids that are embedded into the surface of the solid core during manufacture. Mixtures of two or more phospholipids can be used to vary the surface properties and reactivity of the liposphere. In some embodiments the phospholipid may be selected to enhance adhesion of the lipospheres to the surfaces to which they are applied, or agents which they are used to deliver.

### Phospholipid

A phospholipid is a phosphorylated diacylglyceride molecule or its derivative. The parent structure is diacylglycerol phosphate, or phosphatidic acid. Phosphatidyl choline (lecithin) is the choline ester of phosphorylated diacylglyceride. Synthetic lecithin are available with acyl chain lengths ranging from 4 to 19 carbons. The preferred lecithins for biological applications are those with alkyl chain lengths in the biological range (10 to 18 carbons). Naturally occurring lecithin can be obtained from a variety of sources such as egg, bovine heart, or soy bean. Unsaturated lecithins (dioleoyl; dilinoleoyl; alpha-palmitoyl, beta oleoyl; alpha palmitoyl, beta linoleoyl; and alpha oleoyl, beta palmitoyl), dianachidonyl lecithin (highly unsaturated and a prostaglandin precursor), and alpha palmito beta myristoyl lecithin are also available.

A molecule somewhat structurally related to phosphatidic acid, sphingomyelin, is also suitable for use in the coating of lipospheres.

Certain phospholipids, such as phosphatidic acid, phosphatidyl serine, phosphatidyl inositol, cardiolipin (diphosphatidyl glycerol), and phosphatidyl glycerol, can react with calcium, causing aggregation or the binding of lipospheres to cell membranes. These reactions can be minimized by combining these phospholipids with non-calcium binding phospholipids such as phosphatidyl choline. Phosphatidic acid can be isolated from egg or prepared synthetically (dimyristoyl, dipalmitoyl and distearoyl derivatives are available from Calbiochem). Bovine phosphatidyl serine is also available commercially (Sigma Chemical Co., St. Louis, MO). Phosphatidyl inositol can be isolated from plant or bovine sources. Cardiolipin can be purified from bovine or bacterial sources. Phosphatidyl glycerol can also be purified from bacterial sources or prepared synthetically.

Phosphatidyl ethanolamine in the pure state self-aggregates in a calcium-independent fashion, and is believed to have strong tendencies to aggregate with cell membranes. It should therefore be used in combination with non-aggregating phospholipids. Phosphatidyl ethanolamine is commercially available, isolated from egg, bacteria, bovine, or plasmalogen or as the synthetic dioctadecanoyl, dioleoyl, dihexadecyl, dilauryl, dimyristoyl and dipalmitoyl derivatives.

For cost efficiency, non-injectable food grade lecithins can be used to formulate the lipospheres that are not for use as injectables, such as Centrolex™ or Actiflo™ 70SB, which are lecithins extracted from soya beans manufactured by Central Soya, Ft. Wayne, IN.

### Steroids

Steroids such as cholesterol (a natural constituent of membranes), estrogens (such as estriol, estrone, estradiol and diethylstilbestrol), and androgens (such as androstenedione and testosterone) cannot function alone as the liposphere coating but may be incorporated into the phospholipid surface coating, as well as serve as the core material.

### Amphiphiles

Amphiphiles can be added to the phospholipid coating to alter the surface charge on the liposphere. Examples of amphiphiles that produce a positive charge on the coating are protonated long chain alkyl amines such as stearylamine or the corresponding secondary, tertiary or quaternary substituted amines. Examples of amphiphiles that produce a negative charge are arachidonic acid and other fatty acids.

### Surfactants

The phospholipids can be substituted in part with surfactants such as Tween™ (a hydrophilic class of surfactants), Span™ (a hydrophobic class of surfactants), and polyethylene glycol surfactants.

### Melt preparation of Lipospheres.

In the preferred embodiment, lipospheres are prepared by: (1) melting the agent, or dissolving or dispersing the agent in a liquid vehicle to form a liquid agent which can be solidified by decreasing temperature; (2) adding phospholipid along with an aqueous medium to the liquid agent at a temperature higher than the melting temperature of the liquid agent to form a suspension of the agent; (3) mixing the suspension at a temperature above the melting temperature of the liquid agent until a homogeneous fine preparation is obtained; and then (4) rapidly cooling the preparation to below the melting temperature to solidify the liquid core.

For hydrophilic antigens, the antigen can be dissolved in the aqueous buffer pre-incubated to a temperature higher than the melting temperature of the vehicle and added to the molten mixture of vehicle and phospholipid, followed by shaking until a homogeneous suspension is obtained and rapid cooling of the formulation to below the melting temperature.

Suitable methods of mixing the suspension include mechanical shaking or stirring, fine mixing using homogenizing and sonication.

### Solvent Preparation of Lipospheres.

Alternatively, lipospheres can be prepared by solvent processing. The agent, carrier, and phospholipid are dissolved or mixed in an organic solvent. The solvent is then evaporated, and the resulting solid is mixed with an appropriate amount of buffer and mixing continued until a homogeneous mixture is obtained. The temperature is then reduced to 10°C with continuation mixing for approximately five minutes to form a milky suspension of lipospheres.

For example, an agent, carrier, and phospholipid in an organic solvent are mixed, for example, in ethanol, ethyl acetate, or methylene chloride, added to a round bottomed flask. The solvent is then evaporated, and the resulting solid is mixed with an appropriate amount of buffer and rotation continued until a homogeneous mixture is obtained. The temperature is then reduced to 10°C with continuation rotation for approximately five minutes to form a milky suspension of lipospheres.

In an aqueous solution, the lipospheres form a uniform fine dispersion of microspheres coated with a layer of a phospholipid, with the hydrophobic side of the phospholipid embedded in the outermost layer of the solid hydrophobic core and the hydrophilic side at the aqueous interface.

The particle size, particle distribution, and phospholipid coating can be altered by varying the concentration and properties of the solid vehicle, the lipid, and the mixing method. For intravenous injections, particles should be less than five microns. For subdermal or intramuscular injections, the particle is preferably less than 250 µ in diameter. Larger particle sizes can be used for oral formulations. For controlled drug delivery, the average particle size of the liposphere should be greater than one micron.

The types of biologically active agents that can be incorporated are typified by the following specific examples detailed below relating to incorporation of anesthetic, antigen, and insect control agents.

### Selection of Anesthetic

One of the advantages of the lipospheres containing anesthetic is that they can provide relief for an extended period of time following a single administration, even with anesthetics normally having very short half-lives. The side effects of general anesthesia are avoided. Further, the patient is not burdened with the disorientation and fatigue that accompanies other means of anesthesia. He can often continue with his daily routine. Another advantage of this delivery system is that a vasoconstrictor is not required to attain prolonged duration of analgesic action.

The anesthetic is any compound that is capable of blocking nerve impulses from the area of discomfort to the brain. In a preferred embodiment, the anesthetic is a local anesthetic such as marcaine, procaine (novocaine), chloroprocaine (nesacaine), cocaine, lidocaine, tetracaine (amethocaine, pontocaine), mepivacaine, etidocaine (duranest), bupivacaine (marcaine), dibucaine (cinchocaine, nupercaine), prilocaine (citanest), benzoxinate (dorsacaine), proparacaine (alcaine, opthaine, opthetic), benzocaine (anesthesin), or butamben (butesin).

In an alternative embodiment, a general anesthetic that acts by blocking nerve conduction can be used in the liposphere, for example, one of the halocarbon anesthetics such as halothane, isoflurane, enflurane, or methoxyflurane. Centrally acting narcotics are not suitable for the preparation of lipospheres with local anesthetic activity because they cause a loss of consciousness.

The choice of anesthetic will depend on the type of discomfort to be alleviated and is generally known to those skilled in the art of anesthesia. For example, procaine is commonly injected during dental procedures. Some local anesthetics are too toxic to be given by injection, and are restricted to topical applications to the skin, eye, or mucous membranes. Benoxinate and proparacaine are both commonly applied ophthalmic anesthetics. Cyclomethylcaine is used on damaged or diseased skin and on the mucosa of the rectum and the genitourinary system. Dimethisquin is used as an antipruritic for the relief of itching and pain associated with dermal lesions. Dyclonine, hexylcaine, and pramoxine, are also used to relieve dermal discomfort.

A preferred range of anesthetic to carrier to phospholipid is from 1:0:0.01 to 1:100:100. Other biologically active materials can also be encapsulated in the lipospheres during either melt preparation or solvent preparation, in addition to, or in place of the anesthetics. Examples of other biologically active materials include antiinflammatories, anesthetics, antimicrobials, neuroactive agents, and nutritional supplements. The lipospheres can be used to alleviate the significant discomfort resulting from surgically or trauma induced injuries, including rib and sternum pain associated with thoracic surgery, childbirth, episiotomy, post-surgical incisions, aphthous ulcers, and active herpes lesions, as well as for the control of superficial musculoskeletal pain associated with fractures, dislocations, sprains and strains and the pain associated with dental procedures. The lipospheres can also be used to topically anesthetize the eyes, the skin and mucous membranes.

Lipospheres can be mixed with a variety of other pharmaceutically active agents and/or vehicles for topical, enteral or parenteral administration. The lipospheres can include, or be combined with, other pharmaceutically active agents such as antibiotics, antifungals, antivirals, chemotherapeutic agents, neuroactive compounds, antiinflammatory, anti-arhythmic compounds, anticoagulant, vasoactive compounds or other compounds that would otherwise induce pain. For example, amphotericin B, oil based injectables, and topical creams such as betadine and sulfamefenamide, can be made less painful by administering them in conjunction with anesthetic lipospheres. Liposphere formulations in a paste, suspension, or freeze dried formulation can also contain antimicrobial agents or preservatives (such as gentamicin and vancomycin) to reduce the incidence of local bacterial infections associated with lipid-based injections. Examples of stable preservatives, creams, and ointment formulations are listed in Martindale, The Extra Pharmacopoeia, The Pharmaceutical Press, 28th Edition (1982). The core material must not be soluble in the vehicle.

The lipospheres are administered to the patient topically, enterally (orally, rectally), or parenterally (intravenously, subcutaneously, intramuscularly, intraperitoneally) in the appropriate carrier for administration to the patient. The dosage is determined by the release rate of the anesthetic in combination with the known pharmokinetics of the compound, using standards methods known to those skilled in the art.

The liposphere formulations are stored in aqueous buffer, freeze dried, or in an ointment or cream base, in the freezer, refrigerator or room temperature. It is preferred to store the formulations suspended in an aqueous solution in the refrigerator for immediate use.

### Selection of Antigen

The antigen can be any compound eliciting a desired immunogenic response when administered to an animal in combination with the lipospheres. The antigen may be bacterial, viral, fungal or parasitic in origin, produced naturally and isolated, by recombinant engineering, or chemically synthesized. The bacteria, virus or fungi can be live, attenuated, or killed, administered whole or in part. The antigen may also be a chemical or organic molecule or composition, such as pollen. The antigen may be administered alone or bound to a carrier or chemically modified (for example, chemically crosslinked to form a larger molecule or in combination with an adjuvant. An immunogenic response is characterized as the production of cells having receptors specifically binding to the antigen (T lymphocytes) or cells producing antibodies specifically recognizing the antigen (B lymphocytes).

The amount of antigen incorporated into the lipospheres and administered to an animal can be experimentally determined using standard techniques for administration and measurement of immune response, either cell mediated or antibody titer. As used herein, an antigen is an agent eliciting either, or both, a cell mediated immune response or a humoral immune response.

The lipospheres may also be administered to block an immune response. For example, antibody or antigen may be administered to remove either a circulating antigen, or antibody, from the bloodstream. This is a frequently used technique for desensitization of people with allergies or to inhibit a reaction to a chemical such as an antibiotic or toxin.

For live organisms, special precautions must be taken not to destroy the antigenicity of the organism during the preparation of the liposphere. This can be accomplished, for example, by incorporating the antigen into a carrier having a low melting point such as trimyristin, ethyl stearate, or trilaurin, or by incorporating the virus in a solvent that does not kill or denature the antigenicity of the organism.

The lipospheres are administered to the patient enterally (orally, nasally, rectally), parenterally (intravenously, subcutaneously, intramuscularly, intraperitoneally), transbuccally or transdermally in the appropriate carrier for administration to the patient of an antigen. The dosage is determined by the release rate of the antigen in combination with the immunogenicity of the antigen. A preferred range of antigen to carrier to phospholipid is from 1:0:0.01 to 1:100:100.

The vaccine can be administered in a single dose or in repeated dosages. Alternatively, the core material can be selected to provide release over a sustained period of time, or at a discrete time(s). Mixtures of lipospheres releasing at different discrete times can be used to achieve the same effect as multiple injections. Most vaccines are administered initially two or three times over a two to three month interval to maximize antibody production. The preferred core material for delayed release is a polymer such as polylactide.

### Selection of Adjuvants

The antigen can be incorporated into the solid core alone or in combination with an adjuvant. The core material, the phospholipid, or the entire liposphere may act as an adjuvant. An adjuvant is any compound enhancing the immune response to the antigen, either specifically or non-specifically. Examples of adjuvants are bacteria, either modified live or killed, or immunogenic parts thereof, such as the capsule, alone and in combination with lipid materials. An adjuvant that is routinely used is alum. Examples of adjuvants of bacterial origin are Lipid A and tuberculin bacteria.

### Selection of Repellent.

A number of biologically active agents for control of insects are known and commercially available which are suitable for delivery in lipospheres. The types of insects that can be targeted for control include flying insects such as mosquitos, flies, wasps, yellow jackets, hornets and bees, ants, roaches, lice, fleas, and scabies, as well as many insects that eat or lay their eggs on plants.

A preferred compound for repelling mosquitos and flies is DEET (N,N-diethyl-m-toluamide). A preferred compound for inhibiting insect maturation and reproduction is the insect growth regulator (S)-methoprene [isopropyl (2E,4E,7S)-11-methoxy-3,7,11-trimethyl-2,4-dodecadienoate], marketed as Precor™ by Sandoz Ltd. Preferred compounds for killing fleas are pyrethrins, Piperonyl butoxide, N-octyl bicycloheptene dicarboximide, and 2,3:4,5-Bis (2-butylene) tetrahydro-2-furaldehyde. Other compounds that are marketed for insect control include dimethyl phthalate (DMP), 1,3-ethyl hexanediol (EHD), 1-(3-cyclohexan-1-ylcarbonyl)-2-methylpiperidine (CYM), 1-(3-cyclohexan-1-ylcarbonyl)-2-piperdine (CYP), and phosphorothioic acid O,O-diethyl O-[6-methyl-2-(I-methylethyl)-4-pyrimidinyl] ester, marketed as Diazinon™ by Ortho.

Representative agents to repel insects from plants include plants oils (neem, citronella, eucalyptus, marcosa, turpentine); amines (2-(2-methylamino-ethyl)-pyridine, 3-(methylaminomethyl)-pyridine, cadaverine, 1,8-diaminooctane, spermine, polyethyl eneimine); pesticides (cypermethrin, permethrin, carbofuran, chlormequat, carbendazim, benomyl, fentinhydroxide); carboxylic acids (n-decanoic and dodecanoic), and natural products such as polydogial and azadirctin.

These insect repellents/insecticides/insect growth regulators can be combined with other agents for control of pests, such as rodenticides, or with agents that attract insects, such as pheremones or flavorings, to increase the effectiveness of insecticides or growth regulators. They can be formulated for topical application, for application by spraying, or by application as an aerosol.

These compounds can be combined with other agents such as fertilizers, herbicides (such as atrazine, 1-chloro-4-ethylamino-6-isopropylamino-s-triazine) and fungicides for use in treating or killing plants, topically or systemically. They can also be combined with agents that provide a pleasant smell (such as benzyl acetate, citronellol, rhodinol, phenylethyl alcohol, and bergamet oil) or prevent odor or drying of the skin and hair (such as antiperspirants and deodorants and lotions), for topical application to the skin.

The effective concentration is determined empirically by comparing effectiveness of formulations containing different quantities of active agents. A preferred concentration of lipospheres containing DEET is between 1 and 50% by weight.

### Administration of the Lipospheres containing agent to be delivered or released.

The lipospheres containing the agent to be delivered or released are administered to the environment or the skin of a human or other animal or to plants in a suitable carrier. The lipospheres are preferably administered to the environment by spraying an aqueous dispersion of the lipospheres. The lipospheres are preferably administered to a human or other animal by spraying an aqueous dispersion of lipospheres or by topically applying the lipospheres in an aqueous solution, ointment, cream, or other appropriate carrier. The liposphere formulations are stored in aqueous buffer, freeze dried, or in an ointment or cream base, in the freezer, refrigerator or room temperature, and are stable for an extended period of time.

### Example 1. Method of Preparation of Lidocaine Liposphere with Tristearin Carrier

To a 20 ml vial was added lidocaine (100 mg), tristearin (500 mg), and L-α-lecithin (200 mg). The vial was heated to 65°C to melt the tristearin and dissolve the lidocaine. Not buffer solution (60-70°C, 10 ml; final concentration of lidocaine: 10 mg/ml) was added and the formulation was mixed well by vigorous hand shaking and by vortex for about 5 minutes. The uniform milky like formulation (pH 8.3) was immediately cooled to room temperature by immersing the vial in a dry ice-acetone bath with continued shaking. The pH was adjusted to 7.4 with a 1 N HCl solution.

The drug concentration in the inner core of the resulting lipospheres was 9.5 mg/ml (95% yield), as determined by UV absorption (at 240 and 260 nm) of a 100 microliter of the formulation dissolved in 2 ml of a ethanol:dichloromethane mixture.

The particle size was determined in three ways. Particle sizes of less than 10 micron were determined using a Coulter Particle Analyzer. Particle size of greater than 10 microns were determined by passing the formulation through a set of sieves in the range of 200 and 38 microns and weighing the particles remaining on each screen after drying. The shape and size was verified by examining the samples under a microscope.

The particles were spherical in shape with an average particle size of approximately 40 microns. The distribution of the particle sizes is set out in Table 1.

**Table 1**

| **Liposphere Particle Size.** | |
|---|---|
| **Particle size (microns)** | **Weight %** |
| >200 | <1 |
| 106-200 | 12 |
| 68-106 | 8 |
| 38-68 | 5 |
| 1-38 | 67 |
| <1 | 7 |

Similar results were obtained when 1 gram of tristearin or 0.4 gram of L-α-lecithin was used.

### Example 2. Method of Preparation of Tristearin Liposphere

The preparation method was identical to that of example 1 but without lidocaine. The particle size and shape of the resulting lipospheres were similar to that of the lipospheres of Example 1.

### Example 3. Method of Preparation of Lidocaine Liposphere With Ethyl Stearate Carrier

To a 20 ml vial was added lidocaine (100 mg), ethyl stearate (500 mg, melting point 34-35°C), and L-α-lecithin (200 mg). The vial was heated to 40°C to melt the ethyl stearate and dissolve the lidocaine. Warm buffer solution (35-40°C, 10 ml; final concentration of lidocaine 10 mg/ml) was added and the formulation was mixed well by vigorous hand shaking and by vortexing for about 5 minutes. The uniform milky-appearing formulation (pH 8.3) was immediately cooled to a temperature below 20°C by immersing the vial in a dry ice-acetone bath with continued shaking. The pH was adjusted to 7.4 with a 1 N HCl solution.

The resulting lipospheres had a drug concentration of 9.8 mg/ml (98% yield). The average particle size was less than 10 microns.

### Example 4. Method of Preparation of Lidocaine Liposphere without Vehicle

To a 20 ml vial was added lidocaine (200 mg) and L-α-lecithin (100 mg from egg yolk). The vial was heated to 70°C to melt the lidocaine and then hot buffer solution (60-70°C, 10 ml) was added. The formulation was mixed well by vigorous hand shaking and by vortexing for about 5 minutes. The uniform milky-appearing formulation (pH 8.1) was immediately cooled to room temperature by immersing the vial in a dry ice-acetone bath with continued shaking. The pH was adjusted to 7.4 with a 1 N HCl solution.

The resulting lipospheres had a drug concentration of 20 mg/ml (100% yield). The particle size ranged from 0.35 ± 0.176 microns.

### Example 5. Method of Preparation of Indomethacin Liposphere with Tristearin Carrier

To a 20 ml vial was added indomethacin (100 mg), tristearin (500 mg), and L-α-lecithin (200 mg). The vial was heated to 65°C to melt the tristearin. The insoluble indomethacin was dispersed uniformly in the melt using a vortex. Hot buffer solution (60-70°C, 10 ml; final concentration of indomethacin 10 mg/ml) was added and the formulation was mixed well by vigorous hand shaking and by vortex for about 5 minutes. The uniform, milky-appearing formulation (pH 7.1) was immediately cooled to room temperature by immersing the vial in a dry ice-acetone bath with continued shaking. The pH was adjusted to 7.4 with 1 N NaOH solution.

The resulting lipospheres had a drug concentration of 9.6 mg/ml (96% yield, concentration added: 10 mg/ml) as determined by UV absorption at 308 and 350 nm of 100 microliters of the formulation dissolved in 2 ml of ethanol:dichloromethane mixture. The particles were spherical in shape with an average particle size of 35 ± 30 microns.

### Example 6. Method of Preparation of Indomethacin Lipospheres with Ethyl Stearate Carrier

The preparation and analytical methods were the same as in Example 5 but ethyl stearate, formulated at 40°C was used instead of tristearin.

The resulting lipospheres had a drug concentration of 9.8 mg/ml (98% yield) and the average particle size was 20 ± 12 microns.

### Example 7. Method of Preparation of Ibuprofen Liposphere without Carrier

The preparation and analytical methods were tile same as in Example 4 but ibuprofen (300 mg), formulated at 70°C was used instead of lidocaine.

The resulting lipospheres had a drug concentration of 30 mg/ml, and the particle size was 0.173 ± 0.048 (50%) and 0.49 ± 0.076 (50%) microns.

### Example 8. Method of Preparation of Ibuprofen Liposphere with Tristearin Carrier

The preparation and analytical methods were the same as in Example 1 but ibuprofen (300 mg) was used instead of lidocaine, formulated at 70°C.

The resulting lipospheres had a drug concentration of 30 mg/ml and the particle size was 30 ± 32 microns.

### Example 9. Method of Preparation of Methotrexate Lipospheres with Tristearin Carrier

The preparation and analytical methods were the same as in Example 5 but methotrexate (100 mg) was used instead of indomethacin.

The resulting lipospheres had a drug concentration of 9.5 mg/ml and a particle size of 20 ± 10 microns.

### Example 10. Method of Preparation of Lidocaine Lipospheres with Polycaprolactone Carrier

The preparation and analytical methods were the same as in Example 1 but polycaprolactone (500 mg, molecular weight 2000) was used instead of tristearin.

The resulting lipospheres had a drug concentration of 9.5 mg/ml and a particle size of less than 10 microns.

### Example 11. Stability of Lipospheres Prepared in Examples 1-9.

The concentration, particle size and distribution of the liposphere formulations prepared in Examples 1-9 were evaluated and then the formulations were stored at 25°C and at 5°C. With the exception of the lipospheres of example 4, after one day a supernatant layer was formed over the lipospheres that could be reconstituted to a uniform milky-appearing formulation by light shaking for a few seconds. After thirty days, less than 3% of the original drug concentration had been lost. The particle size and distribution were similar to the original formulations.

### Example 12. Reconstitution of Liposphere Formulations Prepared in Examples 1-9

The formulations prepared in Examples 1-9 (3 ml) were lyophilized to dryness to form a sticky fluffy cake. The cake weight varied according to the amount of solids added. Reconstitution of the cakes by addition of sterile water (3 ml) followed by vortexing for 1 minute resulted in formulations with drug concentration and particle size similar to the original formulation.

### Example 13. Method of Preparation of Tetracycline Lipospheres with Tristearin/Ethyl Stearate Carrier

Tetracycline (150 mg) was suspended in a 1 ml dichloromethane solution containing 450 mg tristearin, and then cast on a Petri dish. After solvent evaporation, the resulting film was ground and sieved to a particle size of less than 38 microns. The particles (400 mg) were suspended in 300 mg of molten ethyl stearate containing L-α-lecithin (200 mg) at 40°C. A warm phosphate buffer solution at pH 7.4 (10 ml, 40°C) was added and the formulation was mixed well by vigorous hand shaking and by vortexing for about 5 minutes. The uniform, milky-appearing formulation (pH 8.3) was immediately cooled to room temperature by immersing the vial in dry ice-acetone bath with continued shaking. The pH was adjusted to 7.4 with a 1 N HCl solution.

The resulting lipospheres had a drug content of 9 mg/ml and a particle size ranging from 50 ± 40 microns.

### Example 14. Release Rates from Liposphere Formulations.

In vitro experiments to evaluate the release of the drug formulations prepared as in Examples 1, 3, 4, 5, and 6 were carried out using the following procedure.

To standard cellulose dialysis tubing was added 2 ml of the formulation and sealed. The sealed tubes were immersed in 50 ml of phosphate buffer solution pH 7.40 and placed in a 37°C incubator. The amount of drug released after discrete times was determined by UV absorbance at 240 and 260 nm for lidocaine and 308 and 350 nm for indomethacin. The solution was then replaced with fresh buffer solution. The drug content of the formulations before and after release was determined by UV absorption of a solution of 100 microliters of the formulation in 3 ml dichloromethane:ethanol 2:1 v/v mixture. The results are summarized in Table 2.

As seen in Table 2, drug release is characterized by an initial burst of about 50% of the drug content. About 80% of the lidocaine was released within 24 hours. The remaining 20% was not released from the formulation. Indomethacin was released for over 5 days, to a total of 80% of the original concentration. The remaining 20% was not released from the formulation.

**Table 2**

| **In Vitro Drug Release From Liposphere Formulations** | | | | | |
|---|---|---|---|---|---|
| **Time (hours)** | **Drug Released (%)** | | | | |
| | **Indomethacin** | | **Lidocaine** | | |
| | **Ex 5** | **Ex 6** | **Ex 1** | **Ex 3** | **Ex 4** |
| 1 | 21 | 22 | 26 | 32 | 37 |
| 5 | 45 | 42 | 48.7 | 57.8 | 59.7 |
| 10 | 60 | 58 | 56.7 | 66.4 | 66.9 |
| 24 | 65 | 66 | 78 | 80 | 82 |
| 48 | 69 | 70 | 79 | 80 | 82 |
| 72 | 73 | 73 | | | |
| 120 | 82 | 80 | | | |

### Example 15. Anesthetic Activity of Lidocaine Formulations.

Lidocaine formulations were examined for their local anesthetic activity using a Rat Paw Hyperalgesia model, in which a rat is injected with the formulation in the paw. The tolerance to pain caused by increasing weight on the paw was measured with a Randall-Sellito apparatus. The results are expressed as the weight tolerated by the animal divided by 10 (the higher the number, the more tolerant the animal is to pain and therefore the more effective the release of drug from the liposphere).

The experimental protocol is as follows. Animals (six 250-300 gram rats in each group) received drug (200 microliter, subplantar) at time 0 in both hind feet. One hour prior to measurement carrageenan (which includes inflammation and makes the animal's paw more sensitive to pain) was injected subplantar. Pressure to withdrawal was measured with the Randall-Sellito apparatus. One animal was measured at 6 and 24 hours, another at 48 and 72 hours. The results are summarized in Table 3.

The formulations of Examples 3 and 4 were very effective for more than 24 hours. The formulation of Example 1 was very effective for at least 6 hours with some activity after 24 hours. The standard lidocaine solution was effective for about 2 hours.

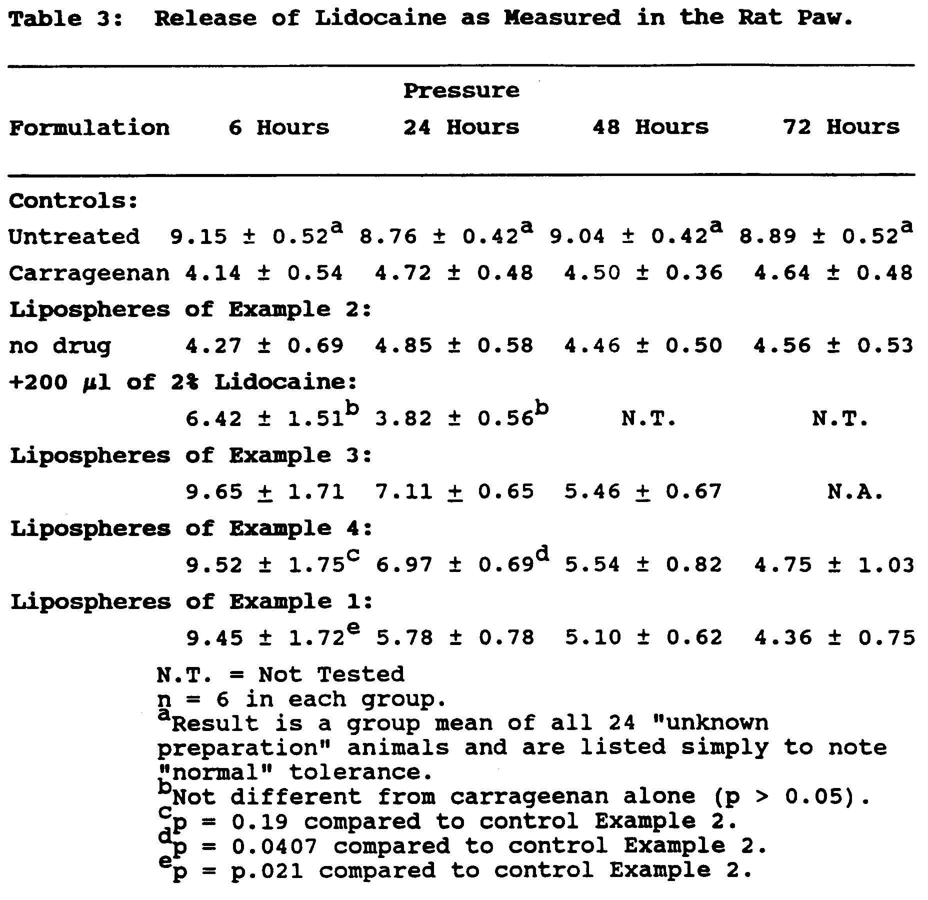

### Example 16. Antiinflammatory Effect of Indomethacin and Ibuprofen Lipospheres

Lipospheres containing indomethacin and ibuprofen were evaluated for their anti-inflammatory effect using a Nsaid Rat Paw Edema model. The results are provided in Table 4.

Animals (6 in each group) received drug intra-peritoneally at t=0. Carrageenan was administered subplantar two hours prior to edema measurement. Each animal could be used for two measurements, one in each foot. Edema was measured with a plethysmometer.

Both indomethacin liposphere formulations were active for more than three days, while the reference indomethacin solution was active for less than 6 hours. The ibuprofen liposphere formulations were active for at least 24 hours.

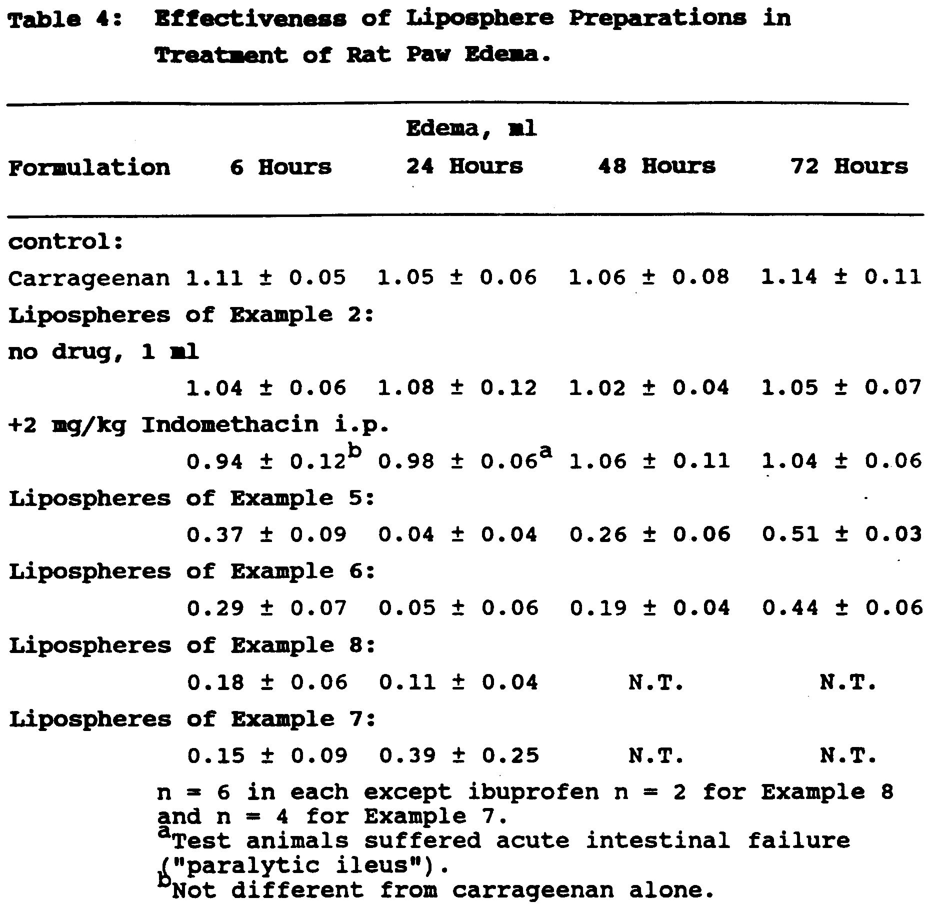

### Example 17: Dexamethasone and Triamcinolone Liposphere Formulations.

Dexamethasone and triamcinolone are relatively water soluble steroids. These drugs were formulated as follows: dexamethasone or triamcinolone powder (100 mg, particle size less than 20 µ) was dispersed in 300 mg molten tristearin and cooled to room temperature. The solid was ground and sieved to particle size of less than 100 µ. The particles (200 mg) were suspended in 200 mg of molten ethyl stearate containing L-α-lecithin (100 mg) at 40°C. Warm phosphate buffer solution, pH 7.4 (5 ml, 40°C) was added and the formulation mixed well by vigorous shaking and by vortexing for about five minutes. The uniform, milky-appearing formulation was immediately cooled to room temperature by immersing the vial in a dry ice - acetone bath with continuous shaking. The preparation was freeze dried for 24 hours and the fluffy, sticky powder was reconstituted before injection. the drug content was 4.7 mg/ml and the particle size was less than 250 microns.

The formulations were both tested for their biological activity using the same experimental model and methods as in example 16. The control consisted of 10 mg drug/ml phosphate buffer. The formulations were active for at least three days, in contrast to the reference solutions which exhibited activity for approximately 24 hours.

### Example 18: Method of Preparation of Lidocaine Liposphere with Tristearin Carrier

To a 20 ml vial was added lidocaine (100 mg), tristearin (500 mg), and L-α-lecithin (200 mg). The vial was heated to 65°C to melt the tristearin and dissolve the lidocaine. 0.1 M phosphate buffer pH 7.4 (60-70°C, 10 ml; final concentration of lidocaine: 10 mg/ml) was added and the formulation mixed well by vigorous hand shaking and by vortexing for about five minutes. The uniform milk-like formulation (pH 8.3) was immediately cooled to room temperature by immersing the vial in a dry ice-acetone bath with continued shaking. The pH was adjusted to 7.4 with 1 N HCl.

The drug concentration in the inner core of the resulting lipospheres was 9.5 mg/ml (95% yield), as determined by UV absorption (at 240 and 260 nm) of 100 microliters of the formulation dissolved in 2 ml of an ethanol:dichloromethane mixture.

The particle size was determined in three ways. Particle sizes of less than 10 micron were determined using a Coulter Particle Analyzer. Particle size of greater than 10 microns were determined by passing the formulation through a set of sieves in the range of 200 to 38 microns and weighing the particles remaining on each screen after drying. The shape and size was verified by examining the samples under a microscope.

The particles were spherical in shape with an average particle size of approximately 40 microns. The distribution of the particle sizes was >200 µ, <1%; 106-200 µ, 12%; 68-106 µ, 8%; 38-68 µ, 5%; 1-38 µ, 67%; and <1 µ, 7%.

Similar results were obtained when 1 gram of tristearin or 0.4 gram of L-α-lecithin was used.

### Example 19: Preparation of Marcaine Liposphere with Ethyl Stearate Carrier.

In a scintillation vial, 100 mg of marcaine was mixed with 400 mg of ethyl stearate. Phosphatidyl choline, PC, (200 mg), from egg yolk or soybean, was spread as a thin film on the side of the scintillation vial. The vial was heated to 60°C, with continuous vortexing, to insure a uniform solid solution. Phosphate buffer solution (45-50°C, 10 ml) was added and the formulation was thoroughly mixed by vortexing. The formulation was immediately brought to room temperature by intermittently dipping the vial in a dry ice-acetone bath with continuous shaking. The formulation was milky in appearance.

The average particle size of the above formulation was 15 microns, as determined by a Coulter Particle Size Analyzer.

### Example 20: Preparation of Marcaine Hydrochloride Liposphere with Ethyl Stearate Carrier.

Lipospheres were made as in Example 19 except that marcaine hydrochloride was used in place of marcaine. The particle size of this formulation was dependent on the initial particle size of the marcaine hydrochloride because it does not melt at the temperature of preparation.

### Example 21: Preparation of Marcaine Liposphere with Tristearin as Carrier.

Lipospheres were made as in Example 19 except that ethyl stearate was substituted with tristearin. The contents of the vial were heated to 65°C. The buffer was heated to 70°C before adding to the solid solution. The lipospheres were spherical in shape and the formulation was milky in appearance.

### Example 22: Comparison of Lipospheres with Different Concentrations of Marcaine.

Marcaine lipospheres were prepared according to the method described in example 19, with different ratios of anesthetic to carrier to phospholipid.

In the first group, the amount of marcaine was varied from 50 mg to 800 mg. The amount of tristearin and phosphatidyl choline remained constant at 400 mg and 200 mg, respectively. In the second group, the amounts of marcaine and phosphatidyl choline were kept constant at 100 mg and 200 mg, respectively, and the amount of tristearin was varied from 50 mg to 800 mg. In the third group, the amounts of marcaine and tristearin were kept constant and the concentration of phosphatidyl choline was varied from 5 to 50 mg/ml.

All of the above liposphere formulations were very uniform and milky in appearance. There was no significant difference between the average particle size of these formulations and the lipospheres described in Example 21.

### Example 23: Preparation of Marcaine Lipospheres with Different Carriers.

Several liposphere formulations were prepared with marcaine:carrier:phosphatidyl choline in a ratio of 1:4:2. The carriers used were tripalmitin (99%), trilaurin, trimyristin, tricaprin and the mixed triglycerides of tristearin and tripalmitin in the ratio of 70:30. The formulations were milky in appearance and had an average particle size of about 15 microns.

### Example 24: Stability of Lipospheres.

The stability of the particle size and the distribution of the liposphere formulations prepared in examples two through six were evaluated by storing the formulations at room temperature and 4°C. The particle sizes and distributions were similar to the original formulation after 30 days. Lipospheres of lidocaine prepared with tristearin as the carrier were stable for ten months at 4°C, and showed no signs of aggregation.

### Example 25: In Vitro Release Rates from Liposphere Formulations.

In vitro release experiments were conducted in dialysis tubing with a molecular weight cut off of 300,000. One milliliter of lipospheres prepared from tristearin and phosphatidyl choline (marcaine:tristearin:phosphatidyl choline, 1:4:2; marcaine:tristearin:phosphatidyl choline, 2:4:2; marcaine:tristearin:phosphatidyl choline, 4:4:2) and marcaine hydrochloride was placed in a prewashed dialysis tubing. The clamped dialysis tubing was placed in a jar containing 800 ml of buffer. The jars were placed on an orbital shaker at 100 rpm, in an oven equilibrated at 37°C. Samples were taken at discrete times and analyzed by HPLC to determine the release kinetics of marcaine from the liposphere formulation. As a control, a solution of marcaine hydrochloride was placed in a dialysis tubing to determine if the dialysis tubing was limiting the rate of release.

Representative release profiles are shown in Figure 1. There is an initial burst of anesthetic released in the first few hours, followed by a sustained release for at least three to four days.

### Example 26: In Vivo Anesthetic Activity of Marcaine Lipospheres.

### A. Effect of concentration of Marcaine.

Liposphere containing either effective concentrations of 10 (Composition A) or 40 mg (Composition B) marcaine were tested in vivo for their local anesthetic activity. Both formulations were prepared as described in Example 21. Composition A was prepared from 1:4:1 marcaine : tristearin : phosphatidyl choline (1:4:1), and composition B was prepared from marcaine : tristearin : phosphatidyl choline (4:4:2).

Male Sprague-Dawley rats (250-300 grams) were used for all studies. A 20% (w:v) suspension of brewer's yeast was made in distilled water. Prior to injection, pain threshold was determined in each animal using a Randall-Sellito apparatus (Stoelting Company, Chicago). This was accomplished by placing the left rear foot on the teflon pad, and applying force to the dorsal surface of the paw at a constant linear rate by means of a second teflon pad. The mass applied to the paw was indicated on a scale. The applied mass at which the animal withdrew its paw was recorded.

To induce hyperalgesia, animals were anesthetized with 5% isoflurane in air. Then 100 µl of the 20% yeast solution was injected after inserting a 5/8 inch 25 g needle through the pad nearest the first digit, in the direction of the center of the foot, to a depth of 6 mm. Animals were immediately returned to their cages, where they recovered from the anesthesia within 3 to 5 minutes. The foot withdrawal score was then measured at the indicated times after the injection, on a scale from 0 to 25 (0 = could not stand any pressure; 25 = could stand extensive pressure). Lipospheres to be tested were co-administered with the yeast solution. Control animals received an identical volume of water. If animals were to be tested more than 24 hours after the administration of lipospheres, then the liposphere solution was injected at time 0 and yeast was injected 24 hours before the measurement was to be made, since yeast-induced hyperalgesia is often not measurable at times greater than 24 hours after administration.

The results are shown in Table 5. The numbers indicate the amount of pressure that the rats could withstand before withdrawing their paws. A test withdrawal value higher than the value measured for yeast alone indicates that the paw is anesthetized. The results demonstrate that the lipospheres effectively release marcaine for at least 48 hours.

**Table 5**

| **Effectiveness of Marcaine Lipospheres in Suppressing Pain** | | | | | |
|---|---|---|---|---|---|
| **Time, Hours** | **0** | **1** | **6** | **24** | **48** |
| Yeast | 5.3±0.3 | 4.6±0.3 | 2.5±0.3 | 2.6±0.4 | 2.3±0.2 |
| Composition A | 5.4±0.3 | 25 ± 0 | 8.2±2.1 | 5.4±0.5 | 4.6±0.2 |
| Composition B | 5.6±4 | 25 ± 0 | 11.4±1.5 | 4.9±0.5 | 5.0±0.3 |

### B. Effect of Carrier

In another in vivo experiment, two different carriers were compared. Formulation A contained marcaine:ethyl stearate:phosphatidyl choline in the ratio of 1:4:2. Formulation B had the same composition except that ethyl stearate was replaced with tristearin. The concentration of marcaine in the final formulation was 10 mg/ml. The results provided in Table 6 demonstrate that marcaine is effectively released for at least 72 hours and that greater release is achieved with ethyl stearate than tristearin.

**Table 6**

| **Effectiveness of Marcaine Lipospheres on Suppressing Pain** | | | | | |
|---|---|---|---|---|---|
| **Time, Hours** | **0** | **6** | **24** | **48** | **72** |
| Carrageenan | 8.2±0.3 | 4.1±0.04 | 4.1±0.04 | 4.1±0.04 | 4.1±0.04 |
| Formulation A | 7.9±1.2 | 8.4±1.9 | 4.4±2.9 | 10.7±1.4 | 9.9±2.8 |
| Formulation B | 7.8±1.2 | 10.7±1.1 | 7.1±0.7 | 5.6±0.8 | 5.5±0.8 |

In another in vivo experiment, the effect of varying the concentration of anesthetic in the liposphere formulation was again evaluated. Several different formulations were prepared as described in Table 7. All of the formulations were prepared as described in Example 20, using 10 ml of buffer.

**Table 7**

| **Marcaine Liposphere Composition** | | | |
|---|---|---|---|
| **Formulation (mgs)** | **Marcaine (mgs)** | **Tristearin(mgs)** | **PCS** |
| A | 100 | 400 | 200 |
| B | 200 | 400 | 200 |
| C | 400 | 400 | 200 |
| D | 600 | 400 | 200 |
| E | 400 | 400 | 400 |

The in vivo anesthesia provided by the above formulations, tested using the method described in-Example 26, is described in Table 8.

**Table 8**

| **Effectiveness of Marcaine Lipospheres in Suppressing Pain** | | | | | |
|---|---|---|---|---|---|
| **Time, Hours** | **0** | **1** | **6** | **24** | **48** |
| Yeast | 4.9±0.3 | 4.6±0.3 | 3.1±0.3 | 2.1±0.4 | 2.4±0.2 |
| A | 4.9±0.3 | 17±2.2 | 8.3±0.8 | 5.3±0.3 | 5.2±0.2 |
| B | 4.8±0.5 | 24.2±0.6 | 11.3±1.8 | 8.0±0.6 | 5.5±0.4 |
| C | 5.2±0.2 | 24±0.2 | 19.2±1.9 | 12.3±2.1 | 11.8±2.0 |
| D | 5.2±0.8 | 25±0.0 | 20.6±1.8 | 15.4±1.5 | 13.8±2.0 |
| E | 5.8±0.4 | 25±0.0 | 21.4±1.3 | 13.6±1.6 | 8.9±0.7 |

The results of Table 8 are shown graphically in Figure 2 as percent effect, where 25 units equals 100%. AL=R

### Example 27: Preparation of Lipospheres by Sonication.

Marcaine was mixed with tristearin in a scintillation vial. The vial was coated with phosphatidyl choline (final formulations of marcaine:tristearin:PCS of 1:4:2 and 1:4:1, corresponding to formulations A and B in Table 8). The vial was heated to 65°C and vortexed for 2 minutes to ensure proper mixing of the two ingredients. To that mixture was then added 10 ml of hot phosphate buffer. The mixture was sonicated for ten minutes with intermittent cooling until it came to room temperature. The lipospheres were less viscous than lipospheres made by the vortex method (Example 19). The average particle size obtained was six microns.

The formulations were tested in vivo for their ability to decrease sensitivity in the rat paw, by the method described in Example 26 above. The results are provided in Table 9.

**Table 9**

| **Effectiveness of Marcaine Lipospheres in Suppressing Pain** | | | | | |
|---|---|---|---|---|---|
| **Time, Hours** | **0** | **1** | **6** | **24** | **48** |
| Yeast | 5.8±0.4 | 5.3±0.3 | 3.7±0.5 | 2.4±0.3 | 2.7±0.2 |
| Formulation A | 6.0±0.4 | 9.5±0.9 | 8.1±1.0 | 7.1±0.5 | 6.2±0.5 |
| Formulation B | 5.8±0.5 | 12.2±1.4 | 5.7±0.4 | 4.6±0.4 | 3.1±0.4 |

### Example 28: Preparation of Lipospheres by Rotoevaporation.

To a round bottom flask containing 100 grams of glass beads (3 mm in diameter), 50 ml of chloroform was added. To that 1 gm of phosphatidyl choline, 1 gm of tristearin and 200 mg of marcaine was added and mixed thoroughly till a clear solution was obtained. The chloroform was evaporated using a rotoevaporizer under reduced temperature at room temperature. The temperature was raised to 40°C after 20 minutes to ensure complete removal of chloroform. A thin film of solids was obtained around the round bottom flask and the glass beads. Ten milliliters of 0.9% saline was added to the round bottom flask and the contents were mixed for 30 minutes at room temperature. At the end of thirty minutes, the temperature was lowered to 10°C by placing in crushed ice and mixing was continued for another half hour. The lipospheres formed were spherical in shape and the average particle size was three to five microns.

Lipospheres of polylactic acid (molecular weight 2,000) were prepared similarly using weight ratio of marcaine:polylactic acid:phosphatidyl choline of 1:4:2. A nice, uniform suspension of lipospheres, having an average diameter of approximately 39 microns, was obtained.

The rate of release of anesthetic from lipospheres prepared as in Example 28 is very similar to the release from lipospheres prepared by vortexing (Example 19). However, the average particle size of liposphere using the method of Example 28 is approximately eight to ten microns and there is a very narrow particle size distribution, whereas the average particle size and distribution of lipospheres prepared by the vortexing method is 15 to 30 microns.

### Example 29: Preparation of Marcaine Lipospheres by Sonication.

A thin film of the mixture of marcaine, tristearin and phosphatidyl choline was prepared in a round bottom flask as described in Example 27. Ten milliliters of isotonic phosphate buffer, pH 7.4 was added to the round bottom flask. An ultrasound probe was inserted into the round bottom flask and the contents were sonicated for 20 minutes at room temperature. The resulting lipospheres were spherical in shape and the average particle size was less than a micron. The lipospheres were exceptionally stable as determined by the particle size analyzer after seven days. The release of marcaine from lipospheres made by sonication is also shown in Figure 1.

### Example 30: Effect of Gamma Radiation Sterilization on Marcaine Lipospheres.

Marcaine lipospheres were prepared with ratios of marcaine:tristearin:phosphatidyl choline (1:4:1 and 2:4:2).

These lipospheres were sent to Isomedix, Inc. (7828 Nagie Avenue, Mountain Grove, Ill., 60053) for gamma irradiation to sterilize the lipospheres. The samples were irradiated for 575 minutes. The maximum dose delivered was 2.33 Mrads. The irradiated samples were analyzed for the particle size distribution, in vitro release characteristics and the in vivo activity.

The average particle size of the irradiated sample was 44 microns. This is not substantially different from the average particle size of samples stored for two weeks at 0°C (or -20°C) in the freezer. The kinetics of release of marcaine from the lipospheres before and after gamma irradiation are provided in Figure 1, along with the data for the release of marcaine from lipospheres prepared as in Example 25.

### Example 31: Treatment or Preparation of Lipospheres with Microfluidizer

Marcaine lipospheres prepared as described in Example 19 had a standard size deviation of 16.90 microns. These lipospheres were pumped into a microfluidizer to refine the particle size. The microfluidizer does not change the particle size but does narrow the particle size distribution. The standard deviation decreased to 10.79 microns after 8 passes and to 9.43 microns after 40 passes. The skewness also decreased considerably. A similar phenomenon was observed when the lipospheres were prepared using a homogenizer.

Lipospheres can also be prepared with a microfluidizer that is equipped with two separate entry ports. The entire equipment is thermally jacketed. Through one entry port, a homogenous melt solution or suspension of drug and carrier is pumped. Through the other port is pumped an aqueous buffer. The two liquids are mixed in the instrument at elevated temperatures where the carrier is melted and rapidly cooled to form the lipospheres. The temperature of the microfluidizer can be changed at any stage of the liposphere processing to manipulate the particle size and distribution.

### Example 32: Sterilization of Lipospheres by Filtration of Lipospheres or Components.

### A. Sterilization by filtration.

In a scintillation vial, 100 mg of marcaine was mixed with 400 mg of ethyl stearate. Phosphatidyl choline, PCS, (200 mg), from egg yolk or soybean, was spread as a thin film on the side of the scintillation vial. The vial was heated to 60°C, with continuous vortexing, to ensure a uniform solid solution. Hot buffer solution (45-50°C, 10 ml) was added and the formulation was thoroughly mixed by vortex. The formulation was filtered through a 0.2 micron membrane filter, using a preheated sterile syringe, into a sterile scintillation vial. The vial was immediately brought to room temperature by intermittently dipping the vial in a dry ice-acetone bath with continuous shaking. The formulation was milky in appearance. The average particle size of this formulation was 15 microns, as determined by LS100 Coulter Particle Size Analyzer.

### B. Sterilization by using presterilized ingredients.

The ingredients are sterilized separately using dry or moist heat, and the final formulation then sterilized with ultra violet radiation. Alternatively, the presterilized ingredients are aseptically processed to obtain sterile lipospheres.

For example, lipospheres were sterilized by filtering the components through 0.2 µ filters. The lipospheres were prepared by dissolving 20 g maracine, 20 g phosphatidyl choline, 1.25 g methyl parabens (0.12%) and 0.5 g propyl parabens (0.05%) in 100% ethanol, filtering, adding 40 g tristearin dissolved in 400 ml boiling 100% ethanol that had also been filtered, and mixing in a one liter round bottom flask. The Mixture was evaporated to dryness by rotoevaporation in a 70°C water bath. 0.1 M phosphate buffer pH 7.4 was added to the dry material up to one liter, then the mixture homogenized five minutes at maximum speed at 70°C, then in dry ice-acetone for another five minutes. The resulting lipospheres were packaged in ten ml vials, stoppered and stored at either 4°C or in the freezer. The average particle diameter was 7.8 µ.

### Example 33: Method of Preparation of Lidocaine Liposphere without Vehicle

To a 20 ml vial was added lidocaine (600 mg) and L-α-lecithin (125 mg from egg yolk). The vial was heated to 70°C to melt the lidocaine and then hot buffer solution (60-70°C, 5 ml) was added. The formulation was mixed well by vigorous hand shaking and by vortexing for about 5 minutes. The uniform milky-appearing formulation was immediately cooled to room temperature by immersing the vial in a dry ice-acetone bath with continued shaking.

The resulting lipospheres had a lidocaine concentration of 125 mg/ml (100% yield). The particle size ranged from 0.35 ± 0.176 microns. The average particle size was 12.63 microns as determined by an LS 100 coulter particle size analyzer. The formulation was effective for 24 hours when tested in the rat paw carrageenan model (Example 16).

### Example 34: Reconstitution of Lyophilized Liposphere Formulations.

Liposphere formulations prepared as described above were lyophilized to dryness to form a sticky fluffy cake. The cake weight varied according to the amount of solids added. Reconstitution of the cakes by addition of sterile water (3 ml) followed by vortexing for one minute resulted in formulations with drug concentration and particle size similar to the original formulation.

### Example 35: Lipospheres Containing Local Anesthetics for severe Pain.

Liposphere formulations, containing 5% and 10% marcaine. Sustained release were prepared from marcaine:tristearin:egg yolk phosphatidyl choline in a ratio of 5:4:2 and 10:4:2. The formulations were uniform, white, and creamy. The lipospheres were tested for their ability to moderate acute and chronic pain. The lipospheres, either in the form of a viscous liquid, gel or solid matrix, were inserted along the sciatic nerve in rats anesthetized with methohexital, chosen for short duration and lack of residual analgesia. Motor block was tested by a standardized scoring procedure when the animals were ambulating. Sensory block was tested via several methods including vocalization threshold to calibrated electrical stimulation via transcutaneous electrodes on the lateral aspect of the foot. Contralateral testing and testing in the saphenous nerve dermatomes were used as controls. Control animals received lipospheres without local anesthetics or sham dissection. In all groups, ipsilateral and contralateral dorsal root ganglia and spinal cord were removed for molecular biology studies as described above. At the end of the study, sciatic nerves were studied in two ways: (1) electrophysiology to confirm reversibility of blockade following washing after removal of the lipospheres, and (2) histopathologic studies of the sciatic nerve to look for signs of injury.

A similar dose of anesthetic in solution would have killed the animals. However, with the liposphere formulation the anesthetic activity remained localized with no systemic effects. Both formulations showed a high degree of motor and sensory block for four days, with better activity at days three and four for the 10% formulation.

The nerve returned to normal after the study with no injury or inflammation as determined by electrophysiology and histopathologic examinations. This study demonstrates the potential use of liposphere-local anesthetics for the management of severe pain, such as intracostal pain.

### Preparation and efficacy of Vaccine Lipospheres.

The preparation and modification of vaccine lipospheres is further understood wwith reference to the following non-limiting examples of the preparation and administration of lipospheres as vaccines.

Examples of antigens used in the following examples include antigens used in malarial vaccines, such as antigens incorporating NANP epitopes from repeat region of circumsporozoite protein of Plasmodium falciparum sporozoite, such as R32NS1, and antigens used in vaccines against Gram-negative bacteria, such as Lipid A from lipopolysaccharide of Gram-negative bacteria, for example, Salmonella minnesota R595.

### Example 36: Method of Preparation of Lipospheres Containing the Immunogen Lipid A.

The lipid A used in this study was primarily monophosphoryl lipid A (a glycophospholipid antigen) from Salmonella Minnesota R595, supplied by List Biological Laboratories, Inc., Campbell, CA.

To a 20 ml vial was added ethyl stearate (300 mg, melting point 34-35°C), L-α-lecithin (150 mg), and lipid A (2 mg). The vial was heated to 40°C to melt the ethyl stearate and disperse the lipid A. Warm phosphate buffer (0.1 M PBS pH 7.4 35-40°C, 10 ml) was added and the formulation was mixed well by vigorous hand shaking and by vortexing for about 5 min. The uniform milky-appearing formulation was immediately cooled to a temperature below 20°C by immersing the vial in a dry ice-acetone bath with continued shaking. Sterile lipospheres were prepared by performing all the preparation steps under aseptic conditions and by using only depyrogenated glassware and sterile disposable devices.

### Example 37: Method of Preparation of Lipospheres Containing the Malaria Antigen R32NS1

The malaria antigens employed in this study R32NS1 (immunization methods) and R32LR (used in ELISA only) were from SmithKline Beecham Pharmaceuticals, Swedeland, PA. The antigens consist of 30 repeats (R) of the tetrapeptide Asn-Ala-Asn-Pro (NANP) interspersed with two tetrapeptides Asn-Val-Asp-Pro (NVDP). These are repeats from the immunodominant repeat region of the circumsporozoite (CS) protein of Plasmodium falciparum linked to an 81 amino acid non-structural protein of influenza virus (NS1), in the case of R32NS1, and the first two amino acids leucine and arginine (LR) from a tetracycline resistant gene in the case of R32LR.

### To a 20 ml vial was added ethyl stearate (200 mg) and L-α-lecithin (100 mg). The vial was heated to 40°C to melt the ethyl stearate. Warm PBS buffer solution (pH 7.4, 35-40°C, 10 ml) containing the malaria antigen R32NS1 (0.2 mg/ml) was added and the formulation was mixed well by vigorous hand shaking and by vortexing for about 5 min. The uniform milky-appearing formulation was immediately cooled to a temperature below 20°C by immersing the vial in a dry ice-acetone bath with continued shaking.

### Example 38: Method of Preparation of Lipospheres Containing Two Immunogens of Markedly Different Chemical Structure: R32NS1 (protein antigen) and Lipid A (glycophospholipid antigen).

To a 20 ml vial was added ethyl stearate (300 mg) L-α-lecithin (150 mg) and lipid A (2 mg). The vial was heated to 40°C to melt the ethyl stearate. Warm PBS buffer solution (pH 7.4, 35-40°C, 10 ml) containing the R32NS1 antigen (0.2 mg/m) was added and the formulation was mixed well by vortexing and hand shaking until an homogeneous milky-appearing formulation was obtained, followed by rapid cooling below 20°C by immersing the vial in a dry ice-acetone bath with continued shaking.

### Example 39: Method of Preparation of Lipospheres Containing One or More Antigens Incorporated into Liposomes

Antigens R32NS and lipid A were encapsulated in multilamellar liposomes composed of dimyristoyl phosphatidyl choline (DMPC), dimyristoyl phosphatidyl glycerol (DMPG) and cholesterol (CHO) at a molar ratio of 9:1:7.5 (final R32NS1 and lipid A concentrations of 1 and 0.2 mg/ml respectively) according to the method described by Alving, et al., "Preparation and use of liposomes in immunological studies." In Liposome Technology, Vol. II, G. Gregoriadis, ed., pp. 157-175 (CRC Press, Boca Raton, 1984). To a 20 ml vial was added ethyl stearate (300 mg) and L-α-lecithin (150 mg) and the vial was heated to 40°C to melt the ethyl stearate. Warm liposome dispersion containing the R32NS1 and lipid A immunogens were added and the formulation was shaken and cooled as described in examples one to three.

### Example 40: Enzyme-linked Immunosorbent Assay (ELISA) for Evaluation of the IgG Antibody Activity Obtained After Immunization with R32NS1 Malaria Antigen Encapsulated in Lipospheres.

Wells of polystyrene microtiter plates were coated with R32LR antigen (0.1 µg) in PBS. Approximately 18 h later the contents of the wells were aspirated, filled with blocking buffer (0.5% casein, 0.01% thimerosal, 0.005% phenol red and 1% Tween™ 20 in PBS) and held for 1 h at room temperature. Rabbit sera to be tested was diluted in 0.5% blocking buffer containing 0.025% Tween 20, and aliquots of each dilution added to triplicate wells. After a 2 h incubation at room temperature, the contents of the wells were aspirated, washed three times with PBS-Tween™ 20 (0.05%) and 50 µg horseradish peroxidase-conjugated goat anti-human IgG (diluted 1:1000 in 0.5% blocking buffer containing 0.025% Tween™ 20) were added to each well. After 1 h the contents of the wells were aspirated, the wells were washed three times with the PBS-Tween™ 20 washing solution and 100 µl peroxidase substrate was then added to each well. ELISA activity was measured as absorbance at 405 nm 1 h after addition of peroxidase substrate using an automatic ELISA plate reader. ELISA units were calculated by multiplying the absorbance at 405 mm at a given dilution by the reciprocal of the dilution.

### Example 41: Immunization with Malaria Antigen Encapsulated in Lipospheres.

Four rabbits were immunized intramuscularly and boosted four weeks later with 0.5 to 1 ml lipospheres containing doses of 100 µg R32NS1 malaria antigen and 40 µg lipid A adsorbed with alum (1.0 mg/ml final concentration) as an adjuvant. The animals were bled before primary immunization and after every two weeks and the sera stored at -20°C. The immune response was determined by measuring the antibody titer, as described in Example 40.

The results in Figure 3A show the increase over time of the anti-malaria antigen IgG antibody in individual rabbits. As demonstrated by this figure, encapsulation of the malaria antigen R32NS1 in lipospheres resulted in increased IgG antibody levels in all four rabbits, especially after the boost at 4 weeks. Figure 3B shows the mean IgG antibody levels for 4 rabbits immunized with lipospheres containing the R32NS1 antigen as a function of the reciprocal of serum dilution.

### Example 42: Immunization with Malaria Antigen Encapsulated in Lipospheres: Effect of Lipid A.

Four groups of 3 rabbits each were immunized intramuscularly at 0 and 4 weeks with four different liposphere formulations (between 0.5 and 1 ml) containing malarial antigens, either M1 and M2, containing lipid A, (10 µg) or M3 and M4 lacking lipid A (10 µg), where the antigen was incorporated in the lipospheres from an aqueous buffer solution (M1) or added as a lyophilized powder to the lipid components in the first step of liposphere preparation (M3), at R32NS1 antigen doses of 100 µg adsorbed with alum (1 mg/ml final concentration). The animals were bled and antibody levels determined as described in Examples 40 and 41.

The results obtained at six weeks after primary immunization (2 weeks after boosting at four weeks) are shown in Figure 4A. Higher levels of IgG antibodies were found for lipospheres containing R32NS1 and lipid A as compared to lipospheres containing only R32NS1 and lacking lipid A (M2, M4). Figure 4A also shows that no significant difference (similar geometric means) was observed whether the antigen was incorporated in the lipospheres from an aqueous buffer solution (M1) or added as a lyophilized powder to the lipid components in the first step of liposphere preparation (M3).

### Example 43: Immunization with Malaria Antigen Incorporated in Lipospheres Prepared as Described in Example 40.

Two groups of three rabbits each were immunized at 0 and 4 weeks by intramuscular injections of between 0.5 to 1 ml of lipospheres containing the malaria antigen R32NS1 (100 µg) and lipid A (LM1, 20 µg) or lacking lipid A (LM2). The animals were bled and IgG antibody levels were measured as described in Examples 40 and 44.

As shown in Figure 4B, the IgG antibody levels of individual rabbits were higher for the LM1 lipospheres formulation, containing lipid A as an adjuvant, as compared with the M1-M4 lipospheres, containing alum an adjuvant, and the LM2 lipospheres, not containing adjuvant.

### Example 44: Enzyme-linked Immunosorbent for Evaluation of the IgG Antibody Activity Obtained After Immunization with Lipospheres Containing Lipid A.

Lipid A was coated onto the surface of wells in polystyrene microtiter plates by addition of an ethanolic solution (l µg lipid A/50 µl/well) to the wells, followed by evaporation of the solvent by air under a fume hood. Plates were blocked by addition of PBS containing 10% fetal calf serum (FCS) and incubating the plates for 2 h at room temperature. Well contents were then removed and 50 µl of serum diluted in blocking buffer was added per well (3 replicates) and the plates incubated overnight at 4°C. Plates were then washed 3 times with PBS and 50 µl of goat anti-rabbit IgG (1:500 dilution in blocking buffer, 1 µg/ml) added to the well. Alkaline phosphatase conjugate was added to the wells and incubated 1 h at room temperature. Plates were again washed three times with PBS and 50 µl of the substrate p-nitrophenyl phosphate at 2 mg/ml in diethanolamine buffer was added to the wells and incubated for 1 h at room temperature. Plates were scanned for optical absorbance at 405 nm using an automatic plate reader. Values reported were adjusted by subtracting values in blank wells that lacked both antigen and monoclonal antibody.

The same assay was used to evaluate the IgM antibody levels obtained after immunization with lipospheres containing Lipid A. substituting goat anti-rabbit IgM for the second antibody.

### Example 45: Immunization with Lipid A and R32NS1 Encapsulated in Lipospheres, and measurement of antibody response to the Lipid A.

Four rabbits were immunized with lipospheres containing lipid A (40 µg) and R32NS1 (100 µg) at weeks 0 and again after four weeks, and the individual animal immune response to lipid A determined utilizing the procedure for analyzing IgG and IgM antibodies to lipid A described in Examples 40 and 44.

As shown in Figures 5A and 5B, encapsulation of lipid A in lipospheres resulted in increased mean IgG and IgM antibody activities, especially after the second immunization four weeks after the primary immunization.

### Example 46: Toxicity and Pyrogenicity Test of Lipospheres Containing R32NS1 and Lipid A.

Lipospheres containing R32NS1 (125 µg/dose) and lipid A (60 µg/dose) were injected intramuscularly into 3 rabbits and the rabbits' temperatures measured for two weeks after injection. The animals were also bled daily and whole white and red blood cell counts performed. The results were compared to those observed in rabbits injected with the same dose of free lipid A (60 µg).

Figure 6 shows that the number of total white blood cells remained constant up to ten days after the intramuscular injection of lipospheres containing lipid A (Figure 6A), compared to a pronounced leukopenia observed in rabbits injected with the same amount of free lipid A (Figure 6B). No pyrogenicity was observed after intramuscular injections of lipid A-encapsulated in lipospheres. The mean rabbit body temperature over the first 10 post injection days was 39.5±0.1, compared to 39.8±0.25 in rabbits injected with the same dose of free lipid A.

### Example 47: Comparison of the animal immune response obtained using lipospheres and liposomes as carriers of the same antigens.

Lipospheres containing the malarial antigen R32NS1 and lipid A were prepared as described in example 38. Liposomes containing the malarial antigen R32NS1 (100 µg) and lipid A (40 µg) were prepared as described by Richards, et al., Infection and Immunity, 56:682-686 (1988). Detection of IgG antibody activities against malaria antigen after immunization was performed using the ELISA procedures in examples 40 and 44. Animal immunization protocols were as described in example 41.

Rabbits were immunized with either lipospheres or liposomes. The lipospheres and the liposomes were injected intramuscularly into either of two groups of four rabbits; the rabbits were boosted with a second injection four weeks later. Both preparations also contained alum as an additional adjuvant.

The mean antibody titer for each of the rabbits in the two groups is shown in Figure 7A. Following the second immunization, the antibody titers in the animals that were injected with the lipospheres were much higher than in those injected with the liposomes. Antigen alone or in the presence of adjuvants produces virtually no titer. The liposomes shown here are the most effective form of delivery for producing a malarial vaccine at this time.

Figure 7B shows the individual titers of each animal in the two groups. It is important to note that the liposomes do not produce uniformly high titers. The dotted horizontal line in this figure shows the titer obtained in the one human who was subsequently shown to have been protected against intentional infection. Several of the liposome-treated animals have presumably protective levels of IgG, but several did not. In contrast, all of the liposphere-treated animals had presumably protective levels; none of these animals failed to produce high titers.

### Preparation and efficacy of Insect Repellant Lipospheres.

The method of preparation of lipospheres, and resulting lipospheres containing repellent, described herein is simple and is characterized by high loading, reproducibility, versatility, and stability. The method and compositions are further illustrated by the following non-limiting examples.

Lipospheres encapsulating DEET were prepared. The liposphere formulations are superior to the formulations now being marketed in several respects. For example, the marketed preparations provide relief for only few hours. Moreover, the concentration of DEET in those formulations is extremely high, about 75 percent. This is particularly harmful because DEET is partially absorbed through skin. There have been several reports of adverse systemic effects associated with the use of DEET. The fermulations described herein provide extended relief using low percent incorporation of DEET.

### Example 48: Preparation of Tristearin liposphere sustained release formulations of DEET.

500 mg of DEET was mixed with 1.0 gm of tristearin in a scintillation vial. The scintillation vial was precoated with 500 mg of phosphatidyl choline (PCS). The scintillation vial was heated to 60°C to obtain a uniform solution of DEET and tristearin. 10 ml of 0.1 M phosphate buffer, pH 7.4 was added to the vial and the contents were mixed by vortexing. After obtaining a uniform dispersion, mixing was continued with intermitted cooling in a dry ice/acetone bath. The resulting formulation was a uniform, smooth textured paste.

Other formulations were prepared by varying the ratio of DEET, tristearin and PCS, as shown in Table 10.

### Example 49: Preparation of Polycaprolactone liposphere sustained release formulations of DEET.

500 mg of DEET was mixed with 1.0 gm of polycaprolactone in a scintillation vial. The scintillation vial was precoated with 500 mg of phosphatidyl choline (PCS). the scintillation vial was heated to 60°C to obtain a uniform solution of DEET and polycaprolactone. 10 ml of 0.1 M phosphate buffer, pH 7.4 was added to the vial and the contents were mixed using vortex. After obtaining a uniform dispersion, mixing was continued with intermitted cooling in a dry ice/acetone bath. The resulting formulation was a smooth, uniform textured paste.

### Example 50: Preparation of DEET lipospheres using the solvent method.

To a round bottom flask containing 100 grams of glass beads (3 mm in diameter), 50 ml of chloroform was added. 1 gm of PCS, 1 gm of tristearin and 0.5 g of DEET was added to the flask and mixed thoroughly till a clear solution was obtained. The chloroform was evaporated using a rotoevaporator under reduced temperature at room temperature. The temperature was raised to 40°C after 20 minutes, to ensure complete removal of chloroform. A thin film of solids was obtained around the round bottom flask and the glass beads. Ten milliliter of 0.9% saline was added to the round bottom flask and the contents were mixed for 5 to 10 minutes at room temperature. The temperature was then lowered to 10°C by placing the flask in crushed ice and mixing was continued for another half hour.

The resulting lipospheres were spherical in shape, with an average particle size of between 8 and 15 microns.

### Example 51: Preparation and comparison of appearance of DEET lipospheres having different liposphere formulations.

DEET formulations were prepared using the various compositions at a ratio of 1:2:1 DEET, carrier, and phosphatidyl choline, up to 10% in water. The carriers used were: tripalmitin, beeswax, stearic acid, ethylstearate, and stearyl alcohol. The phosphatidyl cholines used were: lecithin from egg yolk and from soybean, and partially hydrogenated phosphatidyl choline.

All formulations were milky in appearance with an average particle size of 10 to 30 microns.

### Example 52: Preparation of CCP repellent lipospheres.

Lipospheres containing 10% 1-(3-cyclohexen-1-ylcarbonyl)piperidine (CCP) were prepared by the method described in Example 48 or by the method described in Example 50. The lipospheres were either CCP:Tristearin:PCE 1:2:1 or 1:1:1, by weight.

### Example 53: Demonstration of In vivo efficacy of tristearin and polycaprolactone DEET lipospheres.

### Efficacy Test:

In vivo efficacy tests were conducted on human volunteers who had given informed consent. The formulations prepared in examples 48 and 49 were applied to forearms at various doses, and subjects exposed to mosquitos at various time intervals. The experimental environment consisted of a cylindrical chamber of fixed dimensions, equipped with 16 x 18 mesh mosquito netting. The device contained between ten and twelve fasted mosquitoes having access to the skin through the mosquito netting. The forearm was placed on the mosquito netting and the behavior of the insects was observed. The time over which no mosquitos landed on the skin (100% repellency) was the index for determining the effectiveness of the formulation.

The results of the studies on the tristearin and polycaprolactone DEET lipospheres are summarized in Table 10.

**Table 10**

| **Effectiveness of DEET lipospheres**^{**a**} **in repelling insects.** | | | | | |
|---|---|---|---|---|---|
| **DEET % (w/w)** | **Core material % (w/w)** | **PCS % (w/w)** | **DEET applied mg/cm²** | **Area** | **Effectiveness (hrs)** |
| | | | | | |

| **Tristearin** | | | | | |
|---|---|---|---|---|---|
| 5 | 20 | 10 | 0.125 | 4 | 2 |
| 5 | 10 | 5 | 0.125 | 4 | 3.0 |
| 5 | 20 | 10 | 0.125 | 4 | 2.5 |
| 5 | 10* | 5 | 0.125 | 4 | 3.5 |
| 0 | 20 | 10 | 0 | 4 | 0 |
| 10 | 10 | 10 | 0.25 | 4 | 3 |
| 10 | 30 | 10 | 0.25 | 4 | 4 |
| 15 | 30 | 10 | 0.50 | 4 | 4 |

| **Polycaprolactone** | | | | | |
|---|---|---|---|---|---|
| 5 | 10 | 5 | 0.50 | 19.63 | 2.5 |
| 5 | 20 | 10 | 0.50 | 19.63 | 2.2 |
| 5 | 10* | 5 | | | |
| 0 | 20 | 10 | 0 | 19.63 | 0 |
| 15 | 30 | 10 | 0.75 | 19.63 | 5 |

| | | | | | |
|---|---|---|---|---|---|
| a. 2.5 mg/cm² of each formulation applied. | | | | | |

### Example 54: Preparation and efficacy of tristearin-PCE lipospheres containing different DEET concentrations.

Eight formulations containing 5 and 10% w/w DEET (4 of each concentration) were prepared and tested for repellency activity. All formulations contain tristearin as vehicle, egg yolk lecithin (PCE), DEET and phosphate buffer solution pH 7.4 added to 10 ml. The formulations were prepared as described in Example 48 and tested as described in Example 53 and 55. Table 11 summarizes the formulation compositions and efficacy as determined according to example 53.

**Table 11**

| **Comparison of repellency of lipospheres containing either 5% or 10% DEET.** | | | | |
|---|---|---|---|---|
| | **Composition**^{**a**} **(grams)** | | | **Period of Repellency (hours)** |
| | **DEET** | **Tristearin** | **PCE** | |
| A | 1.0^{b} | 1.5 | 0.5 | 5 |
| B | 1.0^{b} | 1 | 1 | 5 |
| C | 1.0^{b} | 2 | 0.5 | > 6 |
| D | 1.0^{b} | 3 | 1 | 4 to 5 |
| E | 0.5 | 0.75 | 0.25 | 2 to 3 |
| F | 0.5 | 0.5 | 0.5 | 2 to 3 |
| G | 0.5 | 1 | 0.25 | 2 to 3 |
| H | 0.5 | 1.5 | 0.5 | 2 to 3 |

| | | | | |
|---|---|---|---|---|
| a. One gram DEET is equal to 10.0% DEET. | | | | |
| b. Testing and results described in examples 54 and 55. | | | | |

### Example 55: Comparison of the effectiveness of DEET lipospheres in repelling two different species of mosquitos.

### Method for testing repellency.

The repellent was applied to four locations on each arm. One ounce cups with screen bottoms containing five avid (displaying host-seeking behavior) mosquitoes, with Aedes aegypti or Anopheles stephensi, were placed on the treated portions of skin. Mosquitoes were also placed on an untreated section of the arm to serve as a control. After a ten minute period the cups were removed and the number of biting mosquitoes (evident by a blood meal) were counted. This procedure was repeated with previously unexposed mosquitoes every fifteen minutes in the first half hour (with Aedes aegypti only) after application and on every half hour thereafter for a total of four hours after application. Both mosquito species tested: Aedes aegypti and Anopheles stephensi are extremely aggressive biters in this test.

The formulations A, B, C, and D, described in Table 11, were applied at 2.5 mg per cm² of skin surface. Formulations A (1.0 g DEET, 1.5 g tristearin, 0.5 g PCE to 10 ml with buffer) and C (1.0 g DEET, 2 g tristearin, 0.5 g PCE to 10 ml with buffer) were uniform in consistency and applied easily, whereas B (1.0 g DEET, 1 g tristearin, 1 g PCE to 10 ml with buffer) and D (1.0 g DEET, 3 g tristearin, 1 g PCE to 10 ml with buffer) were more paste-like and proved difficult to spread.

**Table 12**

| **Comparison of effectiveness of DEET lipospheres against two species of mosquitos.** | | | | | |
|---|---|---|---|---|---|
| **Time after application (min)** | **Total number biting** | | | | |
| | **Aedes aegypti (Anopheles stephensi)** | | | | |
| | **Formulations A** | **B** | **C** | **D** | **Control** |
| 15 min | 0 | 0 | 0 | 0 | 4 |
| 30 | 0 (0) | 0 (0) | 0 (0) | 0 (0) | 5 (0) |
| 60 | 0 (0) | 0 (0) | 0 (0) | 0 (1) | 5 (3) |
| 90 | 0 (0) | 0 (0) | 0 (0) | 0 (1) | 5 (2) |
| 120 | 0 (0) | 0 (0) | 0 (0) | 0 (0) | 5 (3) |
| 150 | 0 (1) | 0 (0) | 0 (0) | 0 (0) | 5 (5) |
| 180 | 0 (1) | 0 (0) | 0 (0) | 0 (0) | 4 (4) |
| 210 | 0 (1) | 1 (1) | 0 (0) | 0 (1) | 4 (2) |
| 240 | 0 (2) | 0 (1) | 0 (2) | 0 (0) | 0 (2) |
| 270 | 0 (2) | 0 (2) | 0 (2) | 0 (1) | 1 (5) |

Tested against Aedes Aegypti the formulations proved to be repellent, based on prevention of biting, for a minimum of 3.5 hours. At this point there was breakthrough biting on formulation B. Beyond this point in time no further biting occurred on treated surfaces, although biting on the untreated surface dropped as well, presumably because this occurred at a time host-seeking behavior often ceases for Aedes aegypti.

Findings for Anopheles stephensi are not as clear cut. Biting on the untreated surface is more erratic and there was early breakthrough biting on some formulations, notably formulation D. By four hours there was breakthrough biting on all formulations. The literature on mosquito repellency has repeatedly reported limited repellency for DEET tested against Anopheline mosquitoes.

In conclusion, these formulations are repellent to Aedes aegypti for a minimum of four to six hours. It is likely that some of these formulations are effective beyond this point. The fact that formulation C was effective against Anopheles stephensi for four hours, combined with the A. aegypti findings suggest that C is the most promising repellent tested at this time.

### Example 56: Preparation of Oxytetracycline lipospheres.

Liposphere formulations containing 5, 8.5, 10 and 15% w/2 oxytetracycline were prepared as follows: 10 grams of tristearin poser were mixed wiht 10 grams of egg yolk lecithin and with 5, 8.5, 10 or 15 grams of oxytetracycline. The mixture was melted in a 70°C water bath and 70°C 0.1 M phosphate buffer pH 7.4 was added to form a volume of 100 ml. The mixture was mixed for about 5 minute using an homogenizer, and the uniform hot emulsion was rapidly cooled to room temperature or below while mixing by immersing the flask in an acetone/dry ice bath. Uniform yellow formulations were obtained and are liquids that can be injected using a 22 to 25 gauge needle. The average particle size for the formulation is in the range of 10 to 30 microns. The formulations can be mixed with oxytetracycline hydrochloride solutions when an initial drug loading is desired. The formulations were stored in a tight glass container protected from light at room temperature and were physically stable with almost no change in particle.

### Example 57: Comparison of in vivo efficacy between oxytetracycline lipospheres and conventional oxytetracycline.

Serum oxytetracycline concentrations in birds were compared after i.m. injection of a conventional oxytetracycline preparation (Dabicycline 10%) and the oxytetracycline lipospheres, prepared as described in example 56 (LS100). The dose selected was much higher than the usual dose (5 times higher 100 mg/kg compared to 20 mg/kg) in order to have a follow up of blood levels for a larger period of time.

### Experimental Design.

Forty birds, randomly divided into two groups of 20 birds each, were injected intramuscularly in pectoral muscle. The birds were bled seven times. Ten birds from each group were bled at each bleeding.

The blood samples were allowed to clot for two hours at room temperature, after which the samples were centrifuged and the serum was removed and frozen until assay. On the ninth day five birds from each group were autopsied to study histopathology of injection site.

Group A received 1 ml/kg of Dabicycline 100 (10% oxytetracycline).

Group B received 1.18 ml/kg of LS100 (8.5% oxytetracycline).

The volume (dosage) varied from bird to bird, depending on their individual weight.

### Biological Assay.

The drug levels in blood serum samples were determined using the disk diffusion assay. In this assay, antisera is mixed with agar in a petri dish. Depending on the amount of antibiotic present in the sample placed in a well in the agar, a visible area forms around the well as the antibiotic diffuses out of the well and is precipitated by the antisera. The control was a solution of oxytetracycline in untreated bird serum. The minimum concentration detectable by the assay is 0.2 µg/ml.

### Results.

The mean serum concentrations in µg/ml of oxytetracycline following treatment are shown in Table 13.

Treatment with Dabicycline 100 yielded very high serum levels, 19.9 µg/ml and 15.2 µg/ml, at three hours and six hours following administration, respectively. Dabicycline injected birds maintained serum levels of 2.1 µg/ml and 0.4 µg/ml at 24 hours and 48 hours, respectively.

In contrast, the oxytetracycline lipospheres yielded a low peak value and extended the period of release of the oxytetracycline into the blood. The lipospheres yielded serum levels of 2.8 µg/ml and 1.5 µg/ml at 3 hours and 6 hours and serum levels of 4.1 µg/ml, 1.87 µg/ml and 0.7 µg/ml at 24 hours, 48 hours, and 72 hours, respectively.

Potential clinical and therapeutic values for serum oxytetracycline concentrations for the treatment of organisms sensitive to oxytetracycline are in the range of 0.15 to 1.5 µg/ml.

**Table 13**

| **Mean Oxytetracycline Serum Levels following injection of Oxytetracycline or Oxytetracycline lipospheres.** | | |
|---|---|---|
| **Hours post- injection** | **conventional oxytetracycline (10%)** | **oxytetracycline lipospheres (8.5%)** |
| 3 | 19.9 ± 5.9 | 2.8 ± 3.1 |
| 6 | 15.2 ± 3.4 | 1.53 ± 0.6 |
| 24 | 2.1 ± 1.1 | 4.1 ± 2.0 |
| 48 | 0.4 ± 0.24 | 1.87 ± 0.74 |
| 72 | 0.2 ± 0.25 | 0.7 ± 0.48 |
| 96 | 0.04 ± 0.13 | 0.19 ± 0.16 |

In summary, the conventional oxytetracycline formulation shows an initial burst release at the first six hours with some release at 24 hours. The liposphere formulation released drug in a controlled fashion for 48 hours, with some release up to 96 hours.

## Claims (Claims for the following Contracting State(s): AT, BE, CH, LI, DE, DK, FR, GB, IT, LU, NL, SE)

1. A liposphere comprising
a spherical structure having a diameter of greater than one micron and less than 250 microns, including
a core formed of a hydrophobic material that is a solid at a temperature of 25°C or greater and
a phospholipid coating surrounding the core,
wherein the hydrophobic ends of the phospholipid are embedded in the solid core and the hydrophilic ends of the phospholipid are exposed on the surface of the liposphere.

2. The liposphere of claim 1 further comprising a compound to be delivered or released.

3. The liposphere of claim 2 wherein the solid core includes the compound.

4. The liposphere of claim 2 wherein the solid core is formed by the compound and a vehicle that is a solid at approximately 25°C or greater.

5. The liposphere of claim 2 wherein the compound is incorporated into or attached to the phospholipid.

6. The liposphere of claim 4 wherein the vehicle is selected from the group consisting of natural, regenerated and synthetic waxes, fatty acid esters, high fatty alcohols, solid hydrogenated plant oils, paraffins, hard fat, and biodegradable natural and synthetic polymers.

7. The liposphere of claim 2 wherein the compound is a biologically active agent selected from the group consisting of pharmaceutical compounds for enteral, parenteral or topical administration to humans or other animals, agents for pest control, and agents for agricultural applications.

8. The liposphere of claim 7 wherein the pharmaceutical compound is selected from the group consisting of anesthetics, analgesics, antibiotics, antifungals, antivirals, chemotherapeutic agents, neuroactive compounds, antiinflammatory, anti-arhythmic compounds, anticoagulant, vasoactive compounds, vaccines and combinations thereof.

9. The liposphere of claim 8 wherein the anesthetic is a local anesthetic.

10. The liposphere of claim 9 wherein the local anesthetic is selected from the group consisting of marcaine, procaine, chloroprocaine, cocaine, lidocaine, tetracaine, mepivacaine, etidocaine, bupivacaine, dibucaine, prilocaine, benzoxinate, proparacaine, benzocaine, butamben, and combinations thereof.

11. The liposphere of claim 8 wherein the anesthetic is a compound selected from the group consisting of halothane, isoflurane, enflurane, and methoxyflurane.

12. The liposphere of claim 7 wherein the agents for pest control are selected from the group consisting of insecticides, insect repellents, rodenticides, and insect growth regulators.

13. The liposphere of claim 12 wherein the agent is selected from the group consisting of N,N-diethyl-m-toluamide, (S)-methoprene [isopropyl (2E,4E,7S)-11-methoxy-3,7,11-trimethyl-2,4-dodecadienoate], pyrethrins, piperonyl butoxide, N-octyl bicycloheptene dicarboximide, and 2,3:4,5-Bis (2-butylene) tetrahydro-2-furaldehyde, dimethyl phthalate, 1,3-ethyl hexanediol, 1-(3-cyclohexan-1-ylcarbonyl)-2-methylpiperidine, 1-(3-cyclohexan-1-ylcarbonyl)-2-piperdine, phosphorothioic acid O,O-diethyl O-[6-methyl-2-(I-methylethyl)-4-pyrimidinyl] ester, plants oils, 2-(2-methylamino-ethyl)-pyridine, 3-(methylaminomethyl)-pyridine, cadaverine, 1,8-diaminooctane, spermine, polyethyl eneimine, cypermethrin, permethrin, carbofuran, chlormequat, carbendazim, benomyl, fentinhydroxide, n-decanoic and dodecanoic carboxylic acids, polydogial and azadirctin.

14. The lipospheres of claim 12 further comprising compounds selected from the group consisting of skin supplements, fragrances, antiperspirants and deodorants.

15. The liposphere of claim 13 wherein the agent is N,N-diethyl-m-toluamide in a concentration of between 1 and 50%.

16. The liposphere of claim 7 wherein the agent for agricultural application is selected from the group consisting of nutrients, fungicides, insecticides, plant hormones, and herbicides.

17. The liposphere of claim 7 wherein the agent is an antigen.

18. The liposphere of claim 17 wherein the solid core is formed by the antigen in combination with a carrier.

19. The liposphere of claim 17 wherein antigen is incorporated into or onto the phospholipid layer.

20. The liposphere of claim 17 wherein the liposphere, or components of the liposphere, have adjuvant activity.

21. The liposphere of claim 17 further comprising an adjuvant.

22. The liposphere of claim 21 wherein the adjuvant is derived from or present in a bacterial cell.

23. The liposphere of claim 21 wherein either the antigen or the adjuvant is toxic if administered to an animal when not encapsulated within the liposphere.

24. The liposphere of claim 17 wherein, in the absence of the liposphere, the antigen does not elicit antibody production against the agent from which the antigen is derived.

25. The liposphere of claim 17 wherein the antigen is derived from an infectious agent selected from the group consisting of bacteria, viruses, fungi, and parasites.

26. The liposphere of claim 17 wherein the antigen blocks an immune response when administered to a human or animal.

27. The liposphere of claim 17 wherein the antigen is encapsulated within a liposome.

28. The liposphere of claim 17 wherein there is more than one antigen incorporated into or onto the liposphere.

29. The liposphere of claim 7 further comprising a pharmaceutically acceptable carrier for enteral administration to a patient.

30. The liposphere of claim 7 further comprising a pharmaceutically acceptable carrier for topical administration to a patient.

31. The liposphere of claim 7 further comprising a pharmaceutically acceptable carrier for parenteral administration to a patient.

32. The liposphere of claim 7 wherein the solid core is formed by active agent in combination with a carrier and the ratio of biologically active agent to solid core to phospholipid is between 1:0:0.01 and 1:100:100.

33. The liposphere of claim 7 in a carrier for topical administration to plants.

34. The lipospheres of claim 1 wherein the core material is soluble in an organic solvent.

35. A method of making lipospheres comprising a spherical structure having a diameter of greater than one micron and less than 250 microns, including a core formed of a hydrophobic material that is a solid at a temperature of 25°C or greater, and a phospholipid coating surrounding the core, wherein the hydrophobic ends of the phospholipid are embedded in the solid core and the hydrophilic ends of the phospholipid are exposed on the surface of the liposphere, comprising
forming a non-aqueous liquid of the hydrophobic core material,
adding phospholipid and an aqueous solution to the liquid core material,
mixing the liquid core material and phospholipid until a suspension of lipospheres is formed, and rapidly cooling the liquid core material and phospholipid.

36. The method of claim 35 further comprising sonicating the liquid core material and phospholipid after mixing.

37. The method of claim 36 wherein the liquid core material and phospholipid are heated during mixing.

38. The method of claim 35 wherein the liquid core material includes an organic solvent, further comprising evaporating substantially all of the solvent while mixing the core material and phospholipid.

39. The method of claim 38 further comprising adding the aqueous solution to the evaporated core material-phospholipid mixture and mixing until a suspension of lipospheres is formed.

40. The method of claim 35 further comprising melting the material to form the liquid core material.

41. The method of claim 35 further comprising adding a compound to the core material.

42. The method of claim 35 further comprising adding a compound to the phospholipid.

43. The method of claim 35 further comprising adding a compound to the aqueous solution.

44. A liposphere according to any one of Claims 1 to 34 for use in medicine.

## Claims (Claims for the following Contracting State(s): ES, GR)

1. A method of making lipospheres comprising a spherical structure having a diameter of greater than one micron and less than 250 microns, including a core formed of a hydrophobic material that is a solid at a temperature of 25°C or greater, and a phospholipid coating surrounding the core, wherein the hydrophobic ends of the phospholipid are embedded in the solid core and the hydrophilic ends of the phospholipid are exposed on the surface of the liposphere comprising:
forming a non-aqueous liquid of the hydrophobic core material,
adding phospholipid and an aqueous solution to the liquid core material,
mixing the liquid core material and phospholipid until a suspension of lipospheres is formed, and
rapidly cooling the liquid core material and phospholipid.

2. The method of claim 1 further comprising sonicating the liquid core material and phospholipid after mixing.

3. The method of claim 2 wherein the liquid core material and phospholipid are heated during mixing.

4. The method of claim 1 wherein the liquid core material includes an organic solvent, further comprising evaporating substantially all of the solvent while mixing the core material and phospholipid.

5. The method of claim 4 further comprising adding the aqueous solution to the evaporated core material-phospholipid mixture and mixing until a suspension of lipospheres is formed.

6. The method of claim 1 further comprising melting the material to form the liquid core material.

7. The method of claim 1 further comprising adding a compound to the core material.

8. The method of claim 1 further comprising adding a compound to the phospholipid.

9. The method of claim 1 further comprising adding a compound to the aqueous solution.

10. The method of claim 1 wherein the liposphere further comprises a compound to be delivered or released.

11. The method of claim 10 wherein the solid core includes the compound.

12. The method of claim 10 wherein the solid core is formed by the compound and a vehicle that is a solid at approximately 25°C or greater.

13. The method of claim 10 wherein the compound is incorporated into or attached to the phospholipid.

14. The method of claim 12 wherein the vehicle is selected from the group consisting of natural, regenerated and synthetic waxes, fatty acid esters, high fatty alcohols, solid hydrogenated plant oils, paraffins, hard fat, and biodegradable natural and synthetic polymers.

15. The method of claim 10 wherein the compound is a biologically active agent selected from the group consisting of pharmaceutical compounds for enteral, parenteral or topical administration to humans or other animals, agents for pest control, and agents for agricultural applications.

16. The method of claim 15 wherein the pharmaceutical compound is selected from the group consisting of anesthetics, analgesics, antibiotics, antifungals, antivirals, chemotherapeutic agents, neuroactive compounds, antiinflammatory, antiarhythmic compounds, anticoagulant, vasoactive compounds, vaccines and combinations thereof.

17. The method of claim 16 wherein the anesthetic is a local anesthetic.

18. The method of claim 17 wherein the local anesthetic is selected from the group consisting of marcaine, procaine, chloroprocaine, cocaine, lidocaine, tetracaine, mepivacaine, etidocaine, bupivacaine, dibucaine, prilocaine, benzoxinate, proparacaine. benzocaine, butamben, and combinations thereof.

19. The method of claim 16 wherein the anesthetic is a compound selected from the group consisting of halothane, isoflurane, enflurane, and methoxyflurane.

20. The method of claim 15 wherein the agents for pest control are selected from the group consisting of insecticides, insect repellents, rodenticides, and insect growth regulators.

21. The method of claim 20 wherein the agent is selected from the group consisting of N,N-diethyl-m-toluamide, (S)-methoprene [isopropyl (2E, 4E, 7S)-11-methoxy-3,7,11-trimethyl-2,4-dodecadienoate], pyrethrins, piperonyl butoxide, N-octyl bicycloheptene dicarboximide, and 2,3 : 4,5-Bis (2-butylene) tetrahydro-2-furaldehyde, dimethyl phthalate, 1,3-ethyl hexanediol, 1-(3-cyclohexan-1-ylcarbonyl)-2-methylpiperidine, 1-(3-cyclohexan-1-ylcarbonyl)-2-piperdine, phosphorothioic acid 0,0-diethyl 0-[6-methyl-2-(I-methylethyl)-4-pyrimidinyl] ester, plants oils, 2-(2-methylamino-ethyl)-pyridine, 3-(methylaminomethyl)-pyridine, cadaverine, 1,8-diaminooctane, spermine, polyethyl eneimine, cypermethrin, permethrin, carbofuran, chlormequat, carbendazim, benomyl, fentinhydroxide, n-decanoic and dodecanoic carboxylic acids, polydogial and azadirctin.

22. The method of claim 20 further comprising compounds selected from the group consisting of skin supplements, fragrances, antiperspirants and deodorants.

23. The method of claim 21 wherein the agent is N,N-diethyl-m-toluamide in a concentration of between 1 and 50%.

24. The method of claim 15 wherein the agent for agricultural application is selected from the group consisting of nutrients, fungicides, insecticides, plant hormones, and herbicides.

25. The method of claim 15 wherein the agent is an antigen.

26. The method of claim 25 wherein the solid core is formed by the antigen in combination with a carrier.

27. The method of claim 25 wherein antigen is incorporated into or onto the phospholipid layer.

28. The method of claim 25 wherein the liposphere, or components of the liposphere, have adjuvant activity.

29. The method of claim 25 further comprising an adjuvant.

30. The method of claim 29 wherein the adjuvant is derived from or present in a bacterial cell.

31. The method of claim 29 wherein either the antigen or the adjuvant is toxic if administered to an animal when not encapsulated within the liposphere.

32. The method of claim 25 wherein, in the absence of the liposphere, the antigen does not elicit antibody production against the agent from which the antigen is derived.

33. The method of claim 25 wherein the antigen is derived from an infectious agent selected from the group consisting of bacteria, viruses, fungi, and parasites.

34. The method of claim 25 wherein the antigen blocks an immune response when administered to a human or animal.

35. The method of claim 25 wherein the antigen is encapsulated within a liposome.

36. The method of claim 25 wherein there is more than one antigen incorporated into or onto the liposphere.

37. The method of claim 15 further comprising a pharmaceutically acceptable carrier for enteral administration to a patient.

38. The method of claim 15 further comprising a pharmaceutically acceptable carrier for topical administration to a patient.

39. The method of claim 15 further comprising a pharmaceutically acceptable carrier for parenteral administration to a patient.

40. The method of claim 15 wherein the solid core is formed by active agent in combination with a carrier and the ratio of biologically active agent to solid core to phospholipid is between 1:0:0.01 and 1:100:100.

41. The method of claim 15 in a carrier for topical administration to plants.

42. The method of claim 1 wherein the core material is soluble in an organic solvent.

## Patentansprüche (Patentansprüche für folgende(n) Vertragsstaat(en): AT, BE, CH, LI, DE, DK, FR, GB, IT, LU, NL, SE,)

1. Liposphäre, umfassend eine kugelige struktur eines Durchmessers von großer als 1 Mikron und kleiner als 250 Mikron mit einem aus einem bei einer Temperatur von 25°C oder mehr festen hydrophoben Material gebildeten Kern und einem den Kern umhüllenden Phospholipidüberzug, wobei die hydrophoben Enden des Phospholipids in den festen Kern eingebettet sind und die hydrophilen Enden des Phospholipids auf der Liposphärenoberfläche freiliegen.

2. Liposphäre nach Anspruch 1, weiterhin umfassend eine abzugebende oder freizusetzende Verbindung.

3. Liposphäre nach Anspruch 2, wobei der feste Kern die Verbindung enthält.

4. Liposphäre nach Anspruch 2, wobei der feste Kern aus der Verbindung und einem bei etwa 25°C oder mehr festen Vehikel gebildet ist.

5. Liposphäre nach Anspruch 2, wobei die Verbindung in das Phospholipid eingearbeitet ist oder an diesem haftet.

6. Liposphäre nach Anspruch 4, wobei das Vehikel aus der Gruppe natürliche, regenerierte und synthetische Wachse, Fettsäureester, hohe Fettalkohole, feste hydrierte Pflanzenöle, Paraffine, hartes Fett und biologisch abbaubare natürliche und synthetische Polymere ausgewählt ist.

7. Liposphäre nach Anspruch 2, wobei die Verbindung aus einem biologisch aktiven Mittel, ausgewählt aus der Gruppe Arzneimittelverbindungen zur enteralen, parenteralen oder topischen Verabreichung an Menschen oder sonstige Tiere, Mittel zur Schädlingsbekämpfung und Mittel für landwirtschaftliche Anwendungen besteht.

8. Liposphäre nach Anspruch 7, wobei die Arzneimittelverbindung aus der Gruppe Anästhetika, Analgetika, Antibiotika, Mittel gegen Pilze, Mittel gegen Viren, Chemotherapeutika, neuroaktive Verbindungen, entzündungshemmende Verbindungen, Antiarhythmika, Antikoagulanzien, gefäßaktive Verbindungen, Impfstoffe und Kombinationen derselben ausgewählt ist.

9. Liposphäre nach Anspruch 8, wobei es sich bei dem Anästhetikum um ein Lokalanästhetikum handelt.

10. Liposphäre nach Anspruch 9, wobei das Lokalanästhetikum aus der Gruppe Marcaine, Procaine, Chlorprocaine, Cocaine, Lidocaine, Tetracaine, Mepivacaine, Etidocaine,, Bupivacaine, Dibucaine, Prilocaine, Benzoxinate, Proparacaine, Benzocaine, Butamben und Kombinationen derselben ausgewählt ist.

11. Liposphäre nach Anspruch 8, wobei es sich bei dem Anästhetikum um eine Verbindung aus der Gruppe Halothane, Isoflurane, Enflurane und Methoxyflurane handelt.

12. Liposphäre nach Anspruch 7, wobei die Schädlingsbekämpfungsmittel aus der Gruppe Insektizide, Abwehrmittel gegen Insekten, Mittel gegen Nagetiere und Steuerstoffe für das Insektenwachstum ausgewählt ist.

13. Liposphäre nach Anspruch 12, wobei das Mittel aus der Gruppe N,N-Diethyl-m-toluamid, (S)-Methoprene [Isopropyl(2E,4E,7S))-11-methoxy-3,7,11-trimethyl-2,4-dodecadienoat], Pyrethrine, Piperonylbutoxid, M-Octylbicycloheptendicarboximid und 2,3:4,5-Bis-(2-butylen)-tetrahydro-2-furaldehyd, Dimethylphthalat, 1,3-Ethylhexandiol, 1-(3-Cyclohexan-1-ylcarbonyl)-2-methylpiperidin, 1-(3-Cyclohexan-1-ylcarbonyl)-2-piperidin, Phosphorothioesäure, O,O-Diethyl-O-[6-methyl-2-(I-methylethyl)-4-pyrimidinyl]-ester, Planzenöle, 2-(2-Methylaminoethyl)-pyridin, 3-(Methylaminomethyl)-pyridin, Cadaverin, 1,8-Diaminooctan, Spermin, Polyethylenimin, Cypermethrin, Permethrin, Carbofuran, Chlormequat, Carbendazim, Benomyl, Fentinhydroxid, n-Decansäure und Dodecansäuren, Polydogial und Azadirctin ausgewählt ist.

14. Liposphäre nach Anspruch 12, weiterhin umfassend Verbindungen aus der Gruppe Hautergänzungsstoffe, Aromen, schweißhemmende Mittel und Deodorantien.

15. Liposphäre nach Anspruch 13, wobei es sich bei dem Mittel um N,N-Diethyl-m-tuluamid in einer Konzentration zwischen 1 und 50% handelt.

16. Liposphäre nach Anspruch 7, wobei das Mittel für landwirtschaftliche Anwendungen aus der Gruppe Nährstoffe, Fungizide, Insektizide, Pflanzenhormone und Herbizide ausgewählt ist.

17. Liposphäre nach Anspruch 7, wobei das Mittel aus einem Antigen besteht.

18. Liposphäre nach Anspruch 17, wobei der feste Kern aus dem Antigen in Kombination mit einem Träger gebildet ist.

19. Liposphäre nach Anspruch 17, wobei das Antigen in die Phospholipidschicht eingearbeitet ist oder sich auf dieser befindet.

20. Liposphäre nach Anspruch 17, wobei das Liposphäre (selbst) oder Komponenten desselben Hilfsaktivität aufweist (aufweisen).

21. Liposphäre nach Anspruch 17, weiterhin umfassend ein Hilfsmittel.

22. Liposphäre nach Anspruch 21, wobei das Hilfsmittel von einer Bakterienzelle herrührt oder in einer solchen enthalten ist.

23. Liposphäre nach Anspruch 21, wobei entweder das Antigen oder das Hilfsmittel bei Verabreichung an ein Tier in nicht im Liposphären eingekapselter Form toxisch ist.

24. Liposphäre nach Anspruch 17, wobei das Antigen in Abwesenheit des Liposphären eine Antikörperbildung gegen das Mittel, aus dem das Antigen herrührt, provoziert.

25. Liposphäre nach Anspruch 17, wobei das Antigen von einem infektiösen Material, ausgewählt aus der Gruppe Bakterien, Viren, Pilze und Parasiten herrührt.

26. Liposphäre nach Anspruch 17, wobei das Antigen bei Verabreichung an einen Menschen oder ein Tier eine Immunantwort blockiert.

27. Liposphäre nach Anspruch 17, wobei das Antigen in einem Liposom eingekapselt ist.

28. Liposphäre nach Anspruch 17, wobei mehr als ein Antigen in das Liposphäre eingearbeitet ist oder sich auf diesem befindet.

29. Liposphäre nach Anspruch 7, weiterhin umfassend einen pharmazeutisch akzeptablen Träger zur enterischen Verabreichung an einen Patienten.

30. Liposphäre nach Anspruch 7, weiter umfassend einen pharmazeutisch akzeptablen Träger zur topischen Verabreichung an einen Patienten.

31. Liposphäre nach Anspruch 7, weiter umfassend einen pharmazeutisch akzeptablen Träger für die parenterale Verabreichung an einen Patienten.

32. Liposphäre nach Anspruch 7, wobei der feste Kern aus einem aktiven Mittel in Kombination mit einem Träger gebildet ist und das Verhältnis biologisch aktives Mittel/fester Kern/Phospholipid zwischen 1/0/0,01 und 1/100/100 liegt.

33. Liposphäre nach Anspruch 7 in einem Träger zur topischen Verabreichung an Pflanzen.

34. Liposphäre nach Anspruch 1, wobei das Kernmaterial in einem organischen Lösungsmittel löslich ist.

35. Verfahren zur Herstellung von Liposphären mit kugeliger Struktur eines Durchmessers von größer als 1 Mikron und kleiner als 250 Mikron, umfassend einen aus einem bei einer Temperatur von 25°C oder höher festen hydrophoben Material gebildeten Kern und einen den Kern einhüllenden Phospholipidüberzug, wobei die hydrophoben Enden des Phospholipids in den festen Kern eingebettet sind und die hydrophilen Enden des Phospholipids auf der Liposphärenoberfläche freiliegen, durch zubereiten einer nicht-wäßrigen Flüssigkeit des hydrophoben Kernmaterials, zusetzen eines Phospholipids und einer wäßrigen Lösung zu dem flüssigen Kernmaterial, Vermischen des flüssigen Kernmaterials mit dem Phospholipid bis zur Bildung einer Liposphärensuspension und rasches Abkühlen des flüssigen Kernmaterials und Phospholipids.

36. Verfahren nach Anspruch 35, weiterhin umfassend die Beschallung des flüssigen Kernmaterials und Phospholipids nach dem Vermischen.

37. Verfahren nach Anspruch 36, wobei das flüssige Kernmaterial und das Phospholipid während des Mischens erwärmt werden.

38. Verfahren nach Anspruch 35, wobei das flüssige Kernmaterial ein organisches Lösungsmittel enthält und praktisch das gesamte Lösungsmittel während des Mischens des Kernmaterials mit dem Phospholipid verdampft wird.

39. Verfahren nach Anspruch 38, weiterhin umfassend den Zusatz der wäßrigen Lösung zu dem eingedampften Kernmaterial/Phospholipid-Gemisch und das Vermischen bis zum Erhalt einer Liposphärensuspension.

40. Verfahren nach Anspruch 35, weiterhin umfassend das Aufschmelzen des Materials zur Bildung des flüssigen Kernmaterials.

41. Verfahren nach Anspruch 35, weiterhin umfassend den Zusatz einer Verbindung zu dem Kernmaterial.

42. Verfahren nach Anspruch 35, weiterhin umfassend den Zusatz einer Verbindung zu dem Phospholipid.

43. Verfahren nach Anspruch 35, weiterhin umfassend den Zusatz einer Verbindung zu der wäßrigen Lösung.

44. Liposphäre nach einem der Ansprüche 1 bis 34 zur Verwendung in der Medizin.

## Patentansprüche (Patentansprüche für folgende(n) Vertragsstaat(en): ES, GR)

1. Verfahren zur Herstellung von Liposphären mit kugeliger Struktur eines Durchmessers von größer als 1 Mikron und kleiner als 250 Mikron, umfassend einen aus einem bei einer Temperatur von 25°C oder höher festen hydrophoben Material gebildeten Kern und einen den Kern einhüllenden Phospholipidüberzug, wobei die hydrophoben Enden des Phospholipids in den festen Kern eingebettet sind und die hydrophilen Enden des Phospholipids auf der Liposphärenoberfläche freiliegen, durch Zubereiten einer nicht-wäßrigen Flüssigkeit des hydrophoben Kernmaterials, Zusetzen eines Phospholipids und einer wäßrigen Lösung zu dem flüssigen Kernmaterial, Vermischen des flüssigen Kernmaterials mit dem Phospholipid bis zur Bildung einer Liposphärensuspension und rasches Abkühlen des flüssigen Kernmaterials und Phospholipids.

2. Verfahren nach Anspruch 1, weiterhin umfassend die Beschallung des flüssigen Kernmaterials und Phospholipids nach dem Vermischen.

3. Verfahren nach Anspruch 2, wobei das flüssige Kernmaterial und das Phospholipid während des Mischens erwärmt werden.

4. Verfahren nach Anspruch 1, wobei das flüssige Kernmaterial ein organisches Lösungsmittel enthält und praktisch das gesamte Lösungsmittel während des Mischens des Kernmaterials mit dem Phospholipid verdampft wird.

5. Verfahren nach Anspruch 4, weiterhin umfassend den Zusatz der wäßrigen Lösung zu dem eingedampften Kernmaterial/Phospholipid-Gemisch und das Vermischen bis zum Erhalt einer Liposphärensuspension.

6. Verfahren nach Anspruch 1, weiterhin umfassend das Aufschmelzen des Materials zur Bildung des flüssigen Kernmaterials.

7. Verfahren nach Anspruch 1, weiterhin umfassend den Zusatz einer Verbindung zu dem Kernmaterial.

8. Verfahren nach Anspruch 1, weiterhin umfassend den Zusatz einer Verbindung zu dem Phospholipid.

9. Verfahren nach Anspruch 1, weiterhin umfassend den Zusatz einer Verbindung zu der wäßrigen Lösung.

10. Verfahren nach Anspruch 1, wobei das Liposphäre weiterhin eine abzugebende oder freizusetzende Verbindung enthält.

11. Verfahren nach Anspruch 10, wobei der feste Kern die Verbindung enthält.

12. Verfahren nach Anspruch 10, wobei der feste Kern aus der Verbindung und einem bei etwa 25°C oder höher festen Vehikel gebildet ist.

13. Verfahren nach Anspruch 10, wobei die Verbindung in das Phospholipid eingearbeitet ist oder an diesem haftet.

14. Verfahren nach Anspruch 12, wobei das Vehikel aus der Gruppe natürliche, regenerierte und synthetische Wachse, Fettsäureester, hohe Fettalkohole, feste hydrierte Pflanzenöle, Paraffine, hartes Fett und biologisch abbaubare natürliche und synthetische Polymere ausgewählt ist.

15. Verfahren nach Anspruch 10, wobei die Verbindung aus einem biologisch aktiven Mittel, ausgewählt aus der Gruppe Arzneimittelverbindungen zur enteralen, parenteralen oder topischen Verabreichung an Menschen oder sonstige Tiere, Mittel zur Schädlingsbehämpfung und Mittel für landwirtschaftliche Anwendungen besteht.

16. Verfahren nach Anspruch 15, wobei die Arzneimittelverbindung aus der Gruppe Anästhetika, Analgetika, Antibiotika, Mittel gegen Pilze, Mittel gegen Viren, Chemotherapeutika, neuroaktive Verbindungen, entzündungshemmende Verbindungen, Antiarhythmika, Antikoagulanzien, gefäßaktive Verbindungen, Impfstoffe und Kombinationen derselben ausgewählt ist.

17. Verfahren nach Anspruch 16, wobei es sich bei dem Anästhetikum um ein Lokalanästhetikum handelt.

18. Verfahren nach Anspruch 17, wobei das Lokalanästhetikum aus der Gruppe Marcaine, Procaine, Chlorprocaine, Cocaine, Lidocaine, Tetracaine, Mepivacaine, Etidocaine,, Bupivacaine, Dibucaine, Prilocaine, Benzoxinate, Proparacaine, Benzocaine, Butamben und Kombinationen derselben ausgewählt ist.

19. Verfahren nach Anspruch 16, wobei es sich bei dem Anästhetikum um eine Verbindung aus der Gruppe Halothane, Isoflurane, Enflurane und Methoxyflurane handelt.

20. Verfahren nach Anspruch 15, wobei die Schädlingsbekämpfungsmittel aus der Gruppe Insektizide, Abwehrmittel gegen Insekten, Mittel gegen Nagetiere und Steuerstoffe für das Insektenwachstum ausgewählt ist.

21. Verfahren nach Anspruch 20, wobei das Mittel aus der Gruppe N,N-Diethyl-m-toluamid, (S)-Methoprene [Isopropyl(2E,4E,7S))-11-methoxy-3,7,11-trimethyl-2,4-dodecadienoat], Pyrethrine, Piperonylbutoxid, N-Octylbicycloheptendicarboximid und 2,3:4,5-Bis-(2-butylen)-tetrahydro-2-furaldehyd, Dimethylphthalat, 1,3-Ethylhexandiol, 1-(3-Cyclohexan-1-ylcarbonyl)-2-methylpiperidin, 1-(3-Cyclohexan-1-ylcarbonyl)-2-piperidin, Phosphorothioesäure, O,O-Diethyl-O-[6-methyl-2-(I-methylethyl)-4-pyrimidinyl]-ester, Planzenöle, 2-(2-Methylaminoethyl)-pyridin, 3-(Methylaminomethyl)-pyridin, Cadaverin, 1,8-Diaminooctan, Spermin, Polyethylenimin, Cypermethrin, Permethrin, Carbofuran, Chlormequat, Carbendazim, Benomyl, Fentinhydroxid, n-Decansäure und Dodecansäuren, Polydogial und Azadirctin ausgewählt ist.

22. Verfahren nach Anspruch 20, weiterhin umfassend Verbindungen aus der Gruppe Hautergänzungsstoffe, Aromen, schweißhemmende Mittel und Deodorantien.

23. Verfahren nach Anspruch 21, wobei es sich bei dem Mittel um N,N-Diethyl-m-tuluamid in einer Konzentration zwischen 1 und 50% handelt.

24. Verfahren nach Anspruch 15, wobei das Mittel für landwirtschaftliche Anwendungen aus der Gruppe Nährstoffe, Fungizide, Insektizide, Pflanzenhormone und Herbizide ausgewählt ist.

25. Verfahren nach Anspruch 15, wobei das Mittel aus einem Antigen besteht.

26. Verfahren nach Anspruch 25, wobei der feste Kern aus dem Antigen in Kombination mit einem Träger gebildet ist.

27. Verfahren nach Anspruch 25, wobei das Antigen in die Phospholipidschicht eingearbeitet ist oder sich auf dieser befindet.

28. Verfahren nach Anspruch 25, wobei das Liposphäre (selbst) oder Komponenten desselben Hilfsaktivität aufweist (aufweisen).

29. Verfahren nach Anspruch 25, weiterhin umfassend ein Hilfsmittel.

30. Verfahren nach Anspruch 29, wobei das Hilfsmittel von einer Bakterienzelle herrührt oder in einer solchen enthalten ist.

31. Verfahren nach Anspruch 29, wobei entweder das Antigen oder das Hilfsmittel bei Verabreichung an ein Tier in nicht im Liposphären eingekapselter Form toxisch ist.

32. Verfahren nach Anspruch 25, wobei das Antigen in Abwesenheit des Liposphären eine Antikörperbildung gegen das Mittel, aus dem das Antigen herrührt, provoziert.

33. Liposphäre nach Anspruch 25, wobei das Antigen von einem infektiösen Material, ausgewählt aus der Gruppe Bakterien, Viren, Pilze und Parasiten herrührt.

34. Verfahren nach Anspruch 25, wobei das Antigen bei Verabreichung an einen Menschen oder ein Tier eine Immunantwort blockiert.

35. Verfahren nach Anspruch 25, wobei das Antigen in einem Liposom eingekapselt ist.

36. Verfahren nach Anspruch 25, wobei mehr als ein Antigen in das Liposphäre eingearbeitet ist oder sich auf diesem befindet.

37. Verfahren nach Anspruch 15, weiterhin umfassend einen pharmazeutisch akzeptablen Träger zur enterischen Verabreichung an einen Patienten.

38. Verfahren nach Anspruch 15, weiter umfassend einen pharmazeutisch akzeptablen Träger zur topischen Verabreichung an einen Patienten.

39. Verfahren nach Anspruch 15, weiter umfassend einen pharmazeutisch akzeptablen Träger für die parenterale Verabreichung an einen Patienten.

40. Verfahren nach Anspruch 15, wobei der feste Kern aus einem aktiven Mittel in Kombination mit einem Träger gebildet ist und das Verhältnis biologisch aktives Mittel/fester Kern/Phospholipid zwischen 1/0/0,01 und 1/100/100 liegt.

41. Verfahren nach Anspruch 15 in einem Träger zur topischen Verabreichung an Pflanzen.

42. Verfahren nach Anspruch 1, wobei das Kernmaterial in einem organischen Lösungsmittel löslich ist.

## Revendications (Revendications pour l'(les) Etat(s) contractant(s) suivant(s): AT, BE, CH, LI, DE, DK, FR, GB, IT, LU, NL, SE,)

1. Liposphère comprenant :
une structure sphérique ayant un diamètre supérieur à 1 µm et inférieur à 250 µm, comprenant :
un coeur formé d'un matériau hydrophobe qui est un solide à une température de 25°C ou davantage, et
un enrobage de phospholipide entourant le coeur,
dans laquelle les extrémités hydrophobes du phospholipide sont noyées dans le coeur solide et les extrémités hydrophiles du phospholipide sont exposées à la surface de la liposphère.

2. Liposphère selon la revendication 1, comprenant en outre un composé destiné à être délivré ou libéré.

3. Liposphère selon la revendication 2, dans laquelle le coeur solide comprend le composé.

4. Liposphère selon la revendication 2, dans laquelle le coeur solide est formé par le composé et un véhicule qui est un solide à environ 25°C ou davantage.

5. Liposphère selon la revendication 2, dans laquelle le composé est incorporé dans le phospholipide ou fixé à celui-ci.

6. Liposphère selon la revendication 4, dans laquelle le véhicule est choisi dans l'ensemble constitué par les cires naturelles, régénérées et synthétiques, les esters d'acides gras, les alcools gras supérieurs, les huiles végétales hydrogénées solides, les paraffines, les graisses dures, et les polymères naturels et synthétiques biodégradables.

7. Liposphère selon la revendication 2, dans laquelle le composé est un agent biologiquement actif choisi dans l'ensemble constitué par les composés pharmaceutiques pour administration entérale, parentérale ou locale à des être humains ou à d'autres animaux, les agents de lutte contre les parasites, et les agents destinés à des applications pour l'agriculture.

8. Liposphère selon la revendication 7, dans laquelle le composé pharmaceutique est choisi dans l'ensemble constitué par les anesthésiques, les analgésiques, les antibiotiques, les antifongiques, les antiviraux, les agents chimiothérapeutiques, les composés neuroactifs, les anti-inflammatoires, les anti-arythmiques, les anticoagulants, les vasoactifs, les vaccins et leurs combinaisons.

9. Liposphère selon la revendication 8, dans laquelle l'anesthésique est un anesthésique local.

10. Liposphère selon la revendication 9, dans laquelle l'anesthésique local est choisi dans l'ensemble constitué par la marcaïne, la procaïne, la chloroprocaïne, la cocaïne, la lidocaïne, la tétracaïne, la mépivacaïne, l'étidocaïne, la bupivacaïne, la dibucaïne, la prilocaïne, le benzoxinate, la proparacaïne, la benzocaïne, le butoforme, et leurs combinaisons.

11. Liposphère selon la revendication 8, dans laquelle l'anesthésique est un composé choisi dans l'ensemble constitué par l'halothane, l'isoflurane, l'enflurane et le méthoxyflurane.

12. Liposphère selon la revendication 7, dans laquelle les agents de lutte contre les parasites sont choisis dans l'ensemble constitué par les insecticides, les répulsifs pour insectes, les rodenticides et les régulateurs de prolifération des insectes.

13. Liposphère selon la revendication 12, dans laquelle l'agent est choisi dans l'ensemble constitué par le N,N-diéthyl-m-toluamide, le [isopropyl(2E,4E,7S)-11-méthoxy-3,7,11-triméthyl-2,4-dodécadiénoate] de (S)-méthoprène, les pyréthrines, le butylate de pipéronyle, le N-octyldicycloheptènedicarboximide, et le 2,3:4,5-bis(2-butylène)tétrahydro-2-furaldéhyde, le phtalate de diméthyle, le 1,3-éthylhexanediol, la 1-(3-cyclohexane-1-ylcarbonyl)-2-méthylpipéridine, la 1-(3-cyclohexane-1-ylcarbonyl)-2-pipéridine, le phosphorothioate de O,O-diéthyl-O-[6-méthyl-2-(I-méthyléthyl)-4-pyrimidinyle], les huiles végétales, la 2-(2-méthylaminoéthyl)pyridine, la 3-(méthylaminométhyl)pyridine, la cadavérine, le 1,8-diaminooctane, la spermine, la polyéthylène-imine, la cyperméthrine, la perméthrine, le carbofuranne, le chlorméquat, le carbendazime, le bénomyl, le fentinhydroxyde, l'acide carboxylique n-décanoïque, l'acide carboxylique dodécanoïque, le polydogial et l'azadirctine.

14. Liposphères selon la revendication 12, comprenant en outre des composés choisis dans l'ensemble constitué par les adjuvants pour la peau, les parfums, les antiperspirants et les déodorants.

15. Liposphère selon la revendication 13, dans laquelle l'agent est le N,N-diéthyl-m-toluamide à une concentration comprise entre 1 et 50 %.

16. Liposphère selon la revendication 7, dans laquelle l'agent destiné à une application pour l'agriculture est choisi dans l'ensemble constitué par les nutriments, les fongicides, les insecticides, les hormones végétales et les herbicides.

17. Liposphère selon la revendication 7, dans laquelle l'agent est un antigène.

18. Liposphère selon la revendication 17, dans laquelle le coeur solide est formé par l'antigène en combinaison avec un support.

19. Liposphère selon la revendication 17, dans laquelle l'antigène est incorporé dans ou sur la couche de phospholipide.

20. Liposphère selon la revendication 17, dans laquelle la liposphère, ou des composants de la liposphère, ont une activité d'adjuvant.

21. Liposphère selon la revendication 17, comprenant en outre un adjuvant.

22. Liposphère selon la revendication 21, dans laquelle l'adjuvant dérive d'une cellule bactérienne ou est présent dans une telle cellule.

23. Liposphère selon la revendication 21, dans laquelle l'antigène ou l'adjuvant est toxique si on l'administre à un animal alors qu'il n'est pas encapsulé à l'intérieur de la liposphère.

24. Liposphère selon la revendication 17, dans laquelle, en l'absence de liposphère, l'antigène ne provoque pas la production d'anticorps contre l'agent duquel dérive l'antigène.

25. Liposphère selon la revendication 17, dans laquelle l'antigène dérive d'un agent infectieux choisi dans l'ensemble constitué par les bactéries, les virus, les champignons et les parasites.

26. Liposphère selon la revendication 17, dans laquelle l'antigène bloque une réponse immunitaire quand on l'administre à un être humain ou à un animal.

27. Liposphère selon la revendication 17, dans laquelle l'antigène est encapsulé à l'intérieur d'un liposome.

28. Liposphère selon la revendication 17, dans laquelle il y a plus d'un antigène incorporé dans ou sur la liposphère.

29. Liposphère selon la revendication 7, comprenant en outre un support acceptable en pharmacie pour une administration entérale à un patient.

30. Liposphère selon la revendication 7, comprenant en outre un support acceptable en pharmacie pour une administration locale à un patient.

31. Liposphère selon la revendication 7, comprenant en outre un support acceptable en pharmacie pour une administration parentérale à un patient.

32. Liposphère selon la revendication 7, dans laquelle le coeur solide est formé par un agent actif en combinaison avec un support, et le rapport de l'agent biologiquement actif au coeur solide au phospholipide est compris entre 1/0/0,01 et 1/100/100.

33. Liposphère selon la revendication 7, dans un support pour administration locale à des plantes.

34. Liposphères selon la revendication 1, dans lesquelles le matériau de coeur est soluble dans un solvant organique.

35. Procédé de fabrication de liposphères comprenant une structure sphérique ayant un diamètre supérieur à 1 µm et inférieur à 250 µm, comprenant un coeur formé d'un matériau hydrophobe qui est un solide à une température de 25°C ou davantage, et un enrobage de phospholipide entourant le coeur, dans laquelle les extrémités hydrophobes du phospholipide sont noyées dans le coeur solide et les extrémités hydrophiles du phospholipide sont exposées à la surface de la liposphère,
comprenant
la formation d'un liquide non-aqueux du matériau de coeur hydrophobe,
l'addition de phospholipide et d'une solution aqueuse au matériau de coeur liquide,
le mélange du matériau de coeur liquide et du phospholipide jusqu'à ce que se forme une suspension de liposphères, et
le refroidissement rapide du matériau de coeur liquide et du phospholipide.

36. Procédé selon la revendication 35, comprenant en outre la sonication du matériau de coeur liquide et du phospholipide après mélange.

37. Procédé selon la revendication 36, dans lequel le matériau de coeur liquide et le phospholipide sont chauffés pendant le mélange.

38. Procédé selon la revendication 35, dans lequel, le matériau de coeur liquide comprend un solvant organique, comprenant en outre l'évaporation de pratiquement tout le solvant lors du mélange du matériau de coeur et du phospholipide.

39. Procédé selon la revendication 38, comprenant en outre l'addition de la solution aqueuse au mélange évaporé matériau de coeur-phospholipide et le mélange jusqu'à ce qu'il se forme une suspension de liposphères.

40. Procédé selon la revendication 35, comprenant en outre la fusion du matériau pour former le matériau de coeur liquide.

41. Procédé selon la revendication 35, comprenant en outre l'addition d'un composé au matériau de coeur.

42. Procédé selon la revendication 35, comprenant en outre l'addition d'un composé au phospholipide.

43. Procédé selon la revendication 35, comprenant en outre l'addition d'un composé à la solution aqueuse.

44. Liposphère selon l'une quelconque des revendications 1 à 34, destinée à être utilisée en médecine.

## Revendications (Revendications pour l'(les) Etat(s) contractant(s) suivant(s): ES, GR)

1. Procédé de fabrication de liposphères comprenant une structure sphérique ayant un diamètre supérieur à 1 µm et inférieur à 250 µm, comprenant un coeur formé d'un matériau hydrophobe qui est un solide à une température de 25°C ou davantage, et un enrobage de phospholipide entourant le coeur, dans laquelle les extrémités hydrophobes du phospholipide sont noyées dans le coeur solide et les extrémités hydrophiles du phospholipide sont exposées à la surface de la liposphère,
comprenant
la formation d'un liquide non-aqueux du matériau de coeur hydrophobe,
l'addition de phospholipide et d'une solution aqueuse au matériau de coeur liquide,
le mélange du matériau de coeur liquide et du phospholipide jusqu'à ce que se forme une suspension de liposphères, et
le refroidissement rapide du matériau de coeur liquide et du phospholipide.

2. Procédé selon la revendication 1, comprenant en outre la sonication du matériau de coeur liquide et du phospholipide après mélange.

3. Procédé selon la revendication 2, dans lequel le matériau de coeur liquide et le phospholipide sont chauffés pendant le mélange.

4. Procédé selon la revendication 1, dans lequel le matériau de coeur liquide comprend un solvant organique, comprenant en outre l'évaporation de pratiquement tout le solvant lors du mélange du matériau de coeur et du phospholipide.

5. Procédé selon la revendication 4, comprenant en outre l'addition de la solution aqueuse au mélange évaporé matériau de coeur-phospholipide et le mélange jusqu'à ce qu'il se forme une suspension de liposphères.

6. Procédé selon la revendication 1, comprenant en outre la fusion du matériau pour former le matériau de coeur liquide.

7. Procédé selon la revendication 1, comprenant en outre l'addition d'un composé au matériau de coeur.

8. Procédé selon la revendication 1, comprenant en outre l'addition d'un composé au phospholipide.

9. Procédé selon la revendication 1, comprenant en outre l'addition d'un composé à la solution aqueuse.

10. Procédé selon la revendication 1, dans lequel la liposphère comprend en outre un composé destiné à être délivré ou libéré.

11. Procédé selon la revendication 10, dans lequel le coeur solide comprend le composé.

12. Procédé selon la revendication 10, dans lequel le coeur solide est formé par le composé et un véhicule qui est un solide à environ 25°C ou davantage.

13. Procédé selon la revendication 10, dans lequel le composé est incorporé dans le phospholipide ou fixé à celui-ci.

14. Procédé selon la revendication 12, dans lequel le véhicule est choisi dans l'ensemble constitué par les cires naturelles, régénérées et synthétiques, les esters d'acides gras, les alcools gras supérieurs, les huiles végétales hydrogénées solides, les paraffines, les graisses dures, et les polymères naturels et synthétiques biodégradables.

15. Procédé selon la revendication 10, dans lequel le composé est un agent biologiquement actif choisi dans l'ensemble constitué par les composés pharmaceutiques pour administration entérale, parentérale ou locale à des être humains ou à d'autres animaux, les agents de lutte contre les parasites, et les agents destinés à des applications pour l'agriculture.

16. Procédé selon la revendication 15, dans lequel le composé pharmaceutique est choisi dans l'ensemble constitué par les anesthésiques, les analgésiques, les antibiotiques, les antifongiques, les antiviraux, les agents chimiothérapeutiques, les composés neuroactifs, les anti-inflammatoires, les anti-arythmiques, les anticoagulants, les vasoactifs, les vaccins et leurs combinaisons.

17. Procédé selon la revendication 16, dans lequel l'anesthésique est un anesthésique local.

18. Procédé selon la revendication 17, dans lequel l'anesthésique local est choisi dans l'ensemble constitué par la marcaïne, la procaïne, la chloroprocaïne, la cocaïne, la lidocaïne, la tétracaïne, la mépivacaïne, l'étidocaïne, la bupivacaïne, la dibucaïne, la prilocaïne, le benzoxinate, la proparacaïne, la benzocaïne, le butoforme, et leurs combinaisons.

19. Procédé selon la revendication 16, dans lequel l'anesthésique est un composé choisi dans l'ensemble constitué par l'halothane, l'isoflurane, l'enflurane et le méthoxyflurane.

20. Procédé selon la revendication 15, dans lequel les agents de lutte contre les parasites sont choisis dans l'ensemble constitué par les insecticides, les répulsifs pour insectes, les rodenticides et les régulateurs de prolifération des insectes.

21. Procédé selon la revendication 20, dans lequel l'agent est choisi dans l'ensemble constitué par le N,N-diéthyl-m-toluamide, le [isopropyl(2E,4E,7S)-11-méthoxy-3,7,11-triméthyl-2,4-dodécadiénoate] de (S)-méthoprène, les pyréthrines, le butylate de pipéronyle, le N-octyldicycloheptènedicarboximide, et le 2,3:4,5-bis(2-butylène)tétrahydro-2-furaldéhyde, le phtalate de diméthyle, le 1,3-éthylhexanediol, la 1-(3-cyclohexane-1-ylcarbonyl)-2-méthylpipéridine, la 1-(3-cyclohexane-1-ylcarbonyl)-2-pipéridine, le phosphorothioate de O,O-diéthyl-O-[6-méthyl-2-(I-méthyléthyl)-4-pyrimidinyle], les huiles végétales, la 2-(2-méthylaminoéthyl)pyridine, la 3-(méthylaminométhyl)pyridine, la cadavérine, le 1,8-diaminooctane, la spermine, la polyéthylène-imine, la cyperméthrine, la perméthrine, le carbofuranne, le chlorméquat, le carbendazime, le bénomyl, le fentinhydroxyde, l'acide carboxylique n-décanoïque, l'acide carboxylique dodécanoïque, le polydogial et l'azadirctine.

22. Procédé selon la revendication 20, comprenant en outre des composés choisis dans l'ensemble constitué par les adjuvants pour la peau, les parfums, les anti-perspirants et les déodorants.

23. Procédé selon la revendication 21, dans lequel l'agent est le N,N-diéthyl-m-toluamide à une concentration comprise entre 1 et 50 %.

24. Procédé selon la revendication 15, dans lequel l'agent destiné à une application pour l'agriculture est choisi dans l'ensemble constitué par les nutriments, les fongicides, les insecticides, les hormones végétales et les herbicides.

25. Procédé selon la revendication 15, dans lequel l'agent est un antigène.

26. Procédé selon la revendication 25, dans lequel le coeur solide est formé par l'antigène en combinaison avec un support.

27. Procédé selon la revendication 25, dans lequel l'antigène est incorporé dans ou sur la couche de phospholipide.

28. Procédé selon la revendication 25, dans lequel la liposphère, ou des composants de la liposphère, ont une activité d'adjuvant.

29. Procédé selon la revendication 25, comprenant en outre un adjuvant.

30. Procédé selon la revendication 29, dans lequel l'adjuvant dérive d'une cellule bactérienne ou est présent dans une telle cellule.

31. Procédé selon la revendication 29, dans lequel l'antigène ou l'adjuvant est toxique si on l'administre à un animal alors qu'il n'est pas encapsulé à l'intérieur de la liposphère.

32. Procédé selon la revendication 25, dans lequel, en l'absence de liposphère, l'antigène ne provoque pas la production d'anticorps contre l'agent duquel dérive l'antigène.

33. Procédé selon la revendication 25, dans lequel l'antigène dérive d'un agent infectieux choisi dans l'ensemble constitué par les bactéries, les virus, les champignons et les parasites.

34. Procédé selon la revendication 25, dans lequel l'antigène bloque une réponse immunitaire quand on l'administre à un être humain ou à un animal.

35. Procédé selon la revendication 25, dans lequel l'antigène est encapsulé à l'intérieur d'un liposome.

36. Procédé selon la revendication 25, dans lequel il y a plus d'un antigène incorporé dans ou sur la liposphère.

37. Procédé selon la revendication 15, comprenant en outre un support acceptable en pharmacie pour une administration entérale à un patient.

38. Procédé selon la revendication 15, comprenant en outre un support acceptable en pharmacie pour une administration locale à un patient.

39. Procédé selon la revendication 15, comprenant en outre un support acceptable en pharmacie pour une administration parentérale à un patient.

40. Procédé selon la revendication 15, dans lequel le coeur solide est formé par un agent actif en combinaison avec un support, et le rapport de l'agent biologiquement actif au coeur solide au phospholipide est compris entre 1/0/0,01 et 1/100/100.

41. Procédé selon la revendication 15, dans un support pour administration locale à des plantes.

42. Procédé selon la revendication 1, dans lequel le matériau de coeur est soluble dans un solvant organique.
